(19) **Europäisches Patentamt / European Patent Office / Office européen des brevets**

(11) **EP 4 714 977 A1**

(12) **EUROPEAN PATENT APPLICATION**
published in accordance with Art. 153(4) EPC

(43) Date of publication:
25.03.2026 Bulletin 2026/13

(21) Application number: 24810562.9

(22) Date of filing: 20.05.2024

(51) International Patent Classification (IPC):
$C07K\ 16/28^{(2006.01)}$    $A61P\ 37/06^{(2006.01)}$
$C12N\ 15/85^{(2006.01)}$    $A61K\ 39/00^{(2006.01)}$

(52) Cooperative Patent Classification (CPC):
A61K 39/00; A61P 37/06; C07K 16/28; C12N 15/85

(86) International application number:
PCT/IB2024/054911

(87) International publication number:
WO 2024/241206 (28.11.2024 Gazette 2024/48)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 19.05.2023 KR 20230065319

(71) Applicant: Seoul National University R&DB Foundation
Seoul 08826 (KR)

(72) Inventors:
• CHOI, Kyung Ho
Seoul 02814 (KR)
• KIM, Hyo Ri
Seoul 05065 (KR)
• KIM, Ji Hwan
Seoul 01448 (KR)
• LEE, A Neum
Seoul 01044 (KR)
• CHOI, Eun Young
Seoul 02814 (KR)
• NAM, Giri
Seoul 04314 (KR)

(74) Representative: Schiweck Weinzierl Koch
Patentanwälte Partnerschaft mbB
Ganghoferstraße 68 B
80339 München (DE)

Remarks:
The complete document including Reference Table(s) and the Sequence Listing(s) can be downloaded from the EPO website

(54) **USE OF ANTI-CD3 ANTIBODY FOR SELECTIVELY DEPLETING ACTIVATED T CELLS**

(57) The present invention relates to a monovalent antibody or a monovalent antigen-binding fragment thereof that specifically binds to CD3, wherein the monovalent antibody comprises a heavy chain variable region and a light chain variable region, for use in selective depletion of activated T cells. According to one aspect of the present invention, the monovalent anti-CD3 antibody or monovalent antigen-binding fragment thereof selectively depletes activated T cells while relatively sparing non-activated T cells, and thus can be effectively and usefully used as a T-cell depleting agent or a T-cell immunosuppressive agent. In particular, the monovalent antibody or monovalent antigen-binding fragment thereof may be effectively used for preventing or treating T cell-mediated autoimmune diseases, graft-versus-host disease (GVHD), or organ transplant rejection, or for preventing GVHD-related adverse effects in allogeneic CAR-T cell therapies.

EP 4 714 977 A1

FIG. 19

## Description

## Technical Field

The present invention relates to a monovalent antibody or a monovalent antigen-binding fragment thereof that specifically binds to CD3, wherein the monovalent antibody comprises a heavy chain variable region and a light chain variable region, for use in selective depletion of activated T cells. The monovalent antibody or monovalent antigen-binding fragment thereof that specifically binds to CD3 according to the present invention is useful as a T-cell depleting agent and a T-cell immunosuppressive agent.

## Background Art

T cells are a group of cells that play a central role in antigen-specific immune responses and lead the protection of the human body against infection by mediating immune responses specific to pathogen-derived antigens when pathogens invade the body. However, when T-cell responses are excessively induced, they may conversely cause excessive inflammatory responses in the body and thereby mediate inflammatory diseases. For example, excessive T-cell responses against self-antigens can lead to autoimmune diseases, and T-cell responses against grafts during organ transplantation can result in transplant rejection, ultimately leading to transplant failure. In addition, in anti-tumor allogeneic T-cell therapy in which T cells from another individual are administered to a patient for cancer treatment, allogeneic donor T cells may attack normal tissues of the patient and induce graft-versus-host disease (GVHD), which is accompanied by inflammatory responses.

Accordingly, continuous efforts have been made to suppress T cell-mediated inflammatory responses by developing T cell-specific antibodies to deplete T cells. For example, T cell-depleting antibodies such as anti-CD3 antibodies, anti-thymocyte globulin (ATG), and anti-CD52 antibodies have been investigated, and among these, ATG and anti-CD52 antibodies are currently commercially available and in clinical use. However, these antibodies are known to cause an overall reduction in T cells, thereby increasing the risk of infection. In particular, T cell-depleting antibodies in a full IgG format have been reported to induce excessive activation of T cells prior to their depletion, thereby triggering severe inflammatory responses such as cytokine release syndrome (CRS).

Therefore, rather than a strategy of depleting T cells overall, there is a need to develop a strategy that minimizes the risk of cytokine release syndrome and leaves sufficient T cell reservoir to respond to infections by temporarily depleting only activated T cells (effector T cells) that immediately trigger inflammation.

In this regard, the present inventors prepared anti-CD3 antibodies in various formats and, as a result, discovered that a monovalent antibody, particularly an anti-CD3 antibody in the form of a single-chain variable fragment (scFv), can selectively deplete activated T cells while having relatively little effect on non-activated T cells, thereby completing the present invention.

## Detailed Description of the Invention

## Technical Problem

The present invention aims to provide a pharmaceutical composition for preventing or treating T cell-mediated autoimmune diseases, graft-versus-host diseases, or organ transplant rejection, comprising a monovalent antibody or a monovalent antigen-binding fragment thereof that specifically binds to CD3, wherein the monovalent antibody comprises a heavy chain variable region and a light chain variable region.

In addition, the present invention seeks to provide a pharmaceutical composition for suppressing T-cell immunity or depleting activated T cells, comprising a monovalent antibody or a monovalent antigen-binding fragment thereof that specifically binds to CD3, wherein the monovalent antibody comprises a heavy chain variable region and a light chain variable region.

The present invention is intended to provide a method for suppressing T-cell immunity, comprising administering a monovalent antibody or a monovalent antigen-binding fragment thereof that specifically binds to CD3, wherein the monovalent antibody comprises a heavy chain variable region and a light chain variable region.

The present invention aims to provide a method for depleting TCR (T cell receptor)-positive CAR-T cells, comprising:

(a) down-regulating expression of a T cell receptor (TCR) in T cells;
(b) introducing a chimeric antigen receptor (CAR) into the T cells; and
(c) treating the cells obtained in steps (a) and (b) with a monovalent antibody or a monovalent antigen-binding fragment thereof that specifically binds to CD3, wherein the monovalent antibody comprises a heavy chain variable region and a light chain variable region,

wherein steps (a) and (b) are performed in any order.

The present invention aims to provide a monovalent antibody or a monovalent antigen-binding fragment thereof that specifically binds to CD3, wherein the monovalent antibody comprises a heavy chain variable region and a light chain variable region.

The present invention aims to provide a polynucleotide encoding the monovalent antibody or the monovalent antigen-binding fragment thereof, an expression vector comprising the polynucleotide, and a cell comprising the polynucleotide or the expression vector comprising the polynucleotide.

**Technical Solution**

One aspect of the present invention provides a pharmaceutical composition for preventing or treating T cell-mediated autoimmune diseases, graft-versus-host disease, or organ transplant rejection, comprising a monovalent antibody or a monovalent antigen-binding fragment thereof that specifically binds to CD3, wherein the monovalent antibody comprises a heavy chain variable region and a light chain variable region.

As used herein, the term "antibody" refers to an antibody that specifically binds to a particular antigen, and includes not only a complete antibody form but also antigen-binding fragments of the antibody. A complete antibody has a structure comprising two full-length light chains and two full-length heavy chains, wherein each light chain is linked to a heavy chain by a disulfide bond. Heavy chain constant regions include gamma ($\gamma$), mu ($\mu$), alpha ($\alpha$), delta ($\delta$), and epsilon ($\epsilon$) types, and subclasses thereof include gamma 1 ($\gamma$1), gamma 2 ($\gamma$2), gamma 3 ($\gamma$3), gamma 4 ($\gamma$4), alpha 1 ($\alpha$1), and alpha 2 ($\alpha$2). Light chain constant regions include kappa ($\kappa$) and lambda ($\lambda$) types.

As used herein, the term "antigen-binding fragment" refers to a fragment that retains antigen-binding activity, and includes, but is not limited to, Fab, F(ab'), Fv, scFv, or a single-domain antibody (sdAb). Among antigen-binding fragments, a Fab (fragment antigen-binding) has a structure comprising the variable regions of a light chain and a heavy chain, the constant region of the light chain, and the first constant region (CH1) of the heavy chain, and has one antigen-binding site. A Fab' differs from a Fab in that it has a hinge region comprising one or more cysteine residues at the C-terminus of the CH1 domain of the heavy chain. An Fv is the minimal antigen-binding fragment comprising only the heavy chain variable region and the light chain variable region, and recombinant techniques for producing Fv fragments are disclosed in PCT International Patent Publication Nos. WO 88/10649, WO 88/106630, WO 88/07085, WO 88/07086, and WO 88/09344.

As used herein, the term "heavy chain" refers to a full-length heavy chain and fragments thereof, comprising a variable region domain (VH) of an antibody that includes an amino acid sequence having sufficient variable region sequence to confer antigen specificity, and three constant region domains, CH1, CH2, and CH3. As used herein, the term "light chain" refers to a full-length light chain and fragments thereof, comprising a variable region domain (VL) of an antibody that includes an amino acid sequence having sufficient variable region sequence to confer antigen specificity, and a constant region domain (CL).

As used herein, the term "CDR (complementarity determining region)" refers to an amino acid sequence of a hypervariable region of an immunoglobulin heavy chain and light chain. The heavy chain (HCDR1, HCDR2, and HCDR3) and the light chain (LCDR1, LCDR2, and LCDR3) each comprise three CDRs. The CDRs provide key contact residues for antibody binding to an antigen or epitope.

The antibody that specifically binds to CD3 is an antibody that binds to a CD3 molecule present on the surface of T cells, and the antibody may be any one of a monoclonal antibody, a polyclonal antibody, or a recombinant antibody. In addition, the antibody may be a full-length antibody or an antigen-binding fragment. In this case, the antigen-binding fragment may include a portion of an anti-CD3 antibody that is capable of binding to CD3. The antigen-binding fragment may be Fab, Fab', Fv, scFv, or a single-domain antibody (sdAb).

In the present invention, the antibody or antigen-binding fragment that specifically binds to CD3 may be a monovalent antibody. Specifically, the monovalent antibody may be Fab, Fab', Fv, scFv, or a single-domain antibody (sdAb).

Specifically, among the antigen-binding fragments, the scFv may be in a form bound in the order of a light chain variable region, a linker, and a heavy chain variable region of a fragment that specifically binds to CD3 from its N-terminus toward the C-terminus, or in the order of a heavy chain variable region, a linker, and a light chain variable region of a fragment that specifically binds to CD3. In addition, among the antigen-binding fragments, the Fab may be in a form in which a molecule comprising a light chain variable region and a light chain constant region of an antibody that specifically binds to CD3 is combined with a molecule comprising a heavy chain variable region and a heavy chain constant region of an antibody that specifically binds to CD3.

The monovalent antibody or monovalent antigen-binding fragment thereof that specifically binds to CD3 may comprise a complementarity determining region (CDR), specifically CDR1, CDR2, or CDR3, of a heavy chain variable region and/or a light chain variable region of any one selected from known anti-CD3 antibodies OKT3, UCHT1, Teplizumab, Otelixizumab, Visilizumab, and Foralumab. The known anti-CD3 antibodies are merely exemplary and are not limited thereto, and the antibody or antigen-binding fragment thereof may comprise a combination of CDR1, CDR2, or CDR3 of each of the known anti-CD3 antibodies.

The monovalent antibody or monovalent antigen-binding fragment thereof that specifically binds to CD3 may include a light chain variable region comprising light chain CDR1 (LCDR1), light chain CDR2 (LCDR2), and light chain CDR3 (LCDR3) represented by the amino acid sequences of SEQ ID NOs: 23 to 25, respectively, or a heavy chain variable region comprising heavy chain CDR1 (HCDR1), heavy chain CDR2 (HCDR2), and heavy chain CDR3 (HCDR3) represented by the amino acid sequences of SEQ ID NOs: 26 to 28, respectively. In addition, the monovalent antibody or monovalent antigen-binding fragment thereof may include a light chain variable region comprising the amino acid sequence of SEQ ID NO: 29 and/or a heavy chain variable region comprising the amino acid sequence of SEQ ID NO: 30. In one embodiment of the present invention, it may include a linker between the light chain variable region comprising the amino acid sequence of SEQ ID NO: 29 and the heavy chain variable region comprising the amino acid sequence of SEQ ID NO: 30. The linker may have the amino acid sequence of SEQ ID NO: 1 ($(G4S)_3$ linker), but the type of linker is not limited thereto.

The monovalent antibody or monovalent antigen-binding fragment thereof that specifically binds to CD3 may include a light chain variable region comprising LCDR1, LCDR2, and LCDR3 represented by the amino acid sequences of SEQ ID NOs: 32 to 34, respectively, or a heavy chain variable region comprising HCDR1, HCDR2, and HCDR3 represented by the amino acid sequences of SEQ ID NOs: 35 to 37, respectively. In addition, the monovalent antibody or monovalent antigen-binding fragment thereof may include a light chain variable region comprising the amino acid sequence of SEQ ID NO: 38 and/or a heavy chain variable region comprising the amino acid sequence of SEQ ID NO: 39.

In one embodiment of the present invention, the monovalent anti-CD3 antibody or antigen-binding fragment thereof may be an scFv fragment comprising the amino acid sequences of SEQ ID NOs: 13, 22, 31, and 40, or a Fab fragment comprising the amino acid sequences of SEQ ID NOs: 41 and 42. In addition, the anti-CD3 antibody or antigen-binding fragment thereof may comprise the amino acid sequences of SEQ ID NOs: 13, 22, 31, 40, 41, and 42, fragments thereof, or amino acid sequences having at least 70%, 75%, 80%, 85%, 90%, 95%, 97%, 98%, or 99% or greater sequence identity thereto.

The monovalent anti-CD3 antibody or monovalent antigen-binding fragment thereof may further comprise an immunoglobulin kappa sequence. The immunoglobulin kappa sequence may have the amino acid sequence of SEQ ID NO: 2, and may comprise the amino acid sequence of SEQ ID NO: 2, a fragment thereof, or an amino acid sequence having at least 70%, 75%, 80%, 85%, 90%, 95%, 97%, 98%, or 99% or greater sequence identity thereto. In addition, the monovalent anti-CD3 antibody or monovalent antigen-binding fragment thereof may further comprise a tag sequence. The tag may have the amino acid sequence of SEQ ID NO: 3 or 4, but is not limited thereto.

In the present application, even when it is described as "comprising a gene sequence/amino acid sequence of a specific SEQ ID NO." or "having a gene sequence/amino acid sequence of a specific SEQ ID NO.", it is apparent that a gene sequence/amino acid sequence in which some sequences are deleted, modified, substituted, or added can also be used in the present application if it has the same or corresponding function as that consisting of the gene sequence/amino acid sequence of the corresponding SEQ ID NO. In addition, the terms "gene sequence" and "nucleotide sequence" may be used interchangeably in the present application.

For example, as long as the monovalent anti-CD3 antibody or monovalent antigen-binding fragment thereof has the same or equivalent function, it is apparent that sequences in which meaningless sequences are added to internal regions or termini of the sequence of the corresponding SEQ ID NO, or sequences in which portions of the internal regions or termini of the sequence of the corresponding SEQ ID NO are deleted, also fall within the scope of the present invention.

Homology and identity refer to the degree of relatedness between two given nucleotide sequences and may be expressed as a percentage. The terms "homology" and "identity" may often be used interchangeably.

Whether any two sequences have homology or identity can be determined using known computer algorithms such as the "FASTA" program using default parameters as in Pearson et al (1988) [Proc. Natl. Acad. Sci. USA 85: 2444]. Alternatively, it can be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (This includes the GCG program package (Devereux, J., et al, Nucleic Acids Research 12: 387 (1984)), BLASTP, BLASTN, FASTA (Altschul, S. F., et al, J Molec Biol 215: 403 (1990)); Guide to Huge Computers, Martin J. Bishop, ed., Academic Press, San Diego, 1994, and Carrillo et al. (1988) SIAM J Applied Math 48: 1073). For example, the homology or identity of sequences can be determined using BLAST from the National Center for Biotechnology Information (NCBI) of the United States, or ClustalW.

The antibody or antigen-binding fragment thereof that specifically binds to CD3 may comprise a portion of a bispecific antibody. Specifically, among the binding sites for two different antigens of the bispecific antibody, one antigen-binding site may include a site capable of recognizing and binding to CD3. Specifically, the antibody that specifically binds to CD3 is selected from the group consisting of teclistamab, tebentafusp, blinatumomab, catumaxomab, TNB-486, AMG562, duvortuxizumab, AMG910, pasotuxizumab, HPN424, AMG 160, JNJ-63898081, CC-1, AMG 509, HPN536, odronextamab, epcoritamab, glofitamab, mosunetuzumab, JNJ-75348780, vixtimotamab, AMG 330, REGN4018, AMG199, MGD007, EGFR BAT, AMG596, M701, solitomab, MT110, AMG110, AMG 211, MEDI-565, cibisatamab, tidutamab, talquetamab, RG6194, GBR 1302, M802, runimotamab, GEN1044, GEN1047, PF-07062119, AMG 757, BI 764532, HPN328, Hu3F8-BsAb, GEM3PSCA, IMC-C103C, IMC-F106C, JNJ-70218902, AMG 424, elranatamab, ABBV-383, AMG 420, CC-93269, linvoseltamab, alnuctamab, cevostamab, and AMG 427, wherein the heavy chain variable region and/or the light chain variable region target CD3. The types of bispecific antibodies mentioned above are merely examples of bispecific antibodies containing at least one site capable of binding to CD3, and are not limited thereto.

The monovalent antibody or monovalent antigen-binding fragment thereof that specifically binds to CD3, wherein the monovalent antibody comprises a heavy chain variable region and a light chain variable region, according to the present invention, is capable of inducing death of activated T cells and suppressing alloreactive immune responses.

Alloreactive immune responses may be classified into a reaction in which donor T cells attack normal tissues of a recipient when donor T cells are administered to the recipient (graft-versus-host reaction), and a reaction in which recipient T cells attack donor organs or cells when donor organs or cells are transplanted into the recipient (graft rejection). Since both reactions involve T cell-mediated attack responses resulting from activation of donor or recipient T cells, the monovalent antibody or the monovalent antigen-binding fragment thereof that specifically binds to CD3, wherein the monovalent antibody comprises a heavy chain variable region and a light chain variable region, according to the present invention, can selectively kill activated T cells and thereby suppress such alloreactive immune responses.

The T cell-mediated immune diseases may include T cell-mediated autoimmune diseases, graft-versus-host diseases, or organ transplant rejection.

As used herein, the term "autoimmune disease" refers to a disease resulting from an autoimmune response, which is the result of an inappropriate and excessive response to self-antigens. The autoimmune disease may be selected from the group consisting of rheumatoid arthritis, multiple sclerosis, systemic lupus erythematosus, type 1 diabetes, Crohn's disease, scleroderma, Sjögren's syndrome, psoriasis, inflammatory bowel disease, ulcerative colitis, ankylosing spondylitis, interstitial lung disease, uveitis, optic neuritis, peripheral neuropathies, sarcoidosis, antiphospholipid syndrome, inflammatory myopathies, Behçet's disease, alopecia totalis/universalis, pemphigus vulgaris, myasthenia gravis, Graves' disease, Hashimoto's thyroiditis, Guillain-Barré syndrome, celiac disease, and pernicious anemia, but is not limited thereto. More specifically, the T cell-mediated autoimmune disease may be selected from the group consisting of rheumatoid arthritis, systemic lupus erythematosus, Crohn's disease, multiple sclerosis, lupus nephritis, psoriasis (pSS), primary focal and segmental glomerulosclerosis, and immune thrombocytopenia, but is not limited thereto.

As used herein, the term "graft-versus-host disease (GVHD)" refers to a disease in which, when allogeneic hematopoietic stem cell transplantation is performed in patients with hematologic malignancies, donor T cells present in the co-infused peripheral blood or bone marrow recognize normal tissues of the recipient as targets and induce an immune response. In addition to direct cytolytic attacks, donor T cells are known to induce excessive systemic immune responses by secreting pro-inflammatory and fibrotic cytokines or by promoting the production of autoantibodies. Such graft-versus-host disease includes not only that occurring in allogeneic hematopoietic stem cell transplantation, but also graft-versus-host disease caused by TCR-positive CAR-T cells during allogeneic CAR-T cell therapy, as exemplified in the present invention.

As used herein, the term "organ transplant rejection" refers to a disease in which recipient alloreactive T cells, which recognize a transplanted graft from an organ donor as a target, are activated and attack the transplanted graft, thereby causing necrosis of the transplanted graft.

In the present invention, it was confirmed that the monovalent anti-CD3 antibody is capable of depleting activated T cells not only in vitro but also in vivo, which suggests the potential of the monovalent anti-CD3 antibody to be developed as a therapeutic agent for T cell-mediated inflammatory autoimmune diseases caused by activated T cells. This can be readily anticipated from previous examples in which antibodies for T-cell depletion or suppression have been commercially developed with autoimmune diseases such as multiple sclerosis as indications (e.g., alemtuzumab (anti-CD52 antibody) and daclizumab (anti-CD25 antibody)).

In addition, since an anti-CD3 antibody (Teplizumab) has recently begun to be marketed under US FDA approval with an indication for autoimmune diabetes (type 1 diabetes), the monovalent anti-CD3 antibody according to the present invention may be developed as an immunosuppressive agent for T-cell depletion and inactivation. However, bivalent full IgG anti-CD3 antibodies, such as teplizumab, are structurally different from the monovalent anti-CD3 antibody of the present invention, and the monovalent anti-CD3 antibody exhibits significantly enhanced T-cell killing activity compared to bivalent full IgG antibodies. Accordingly, the monovalent anti-CD3 antibody is also functionally distinguishable from bivalent full IgG antibodies.

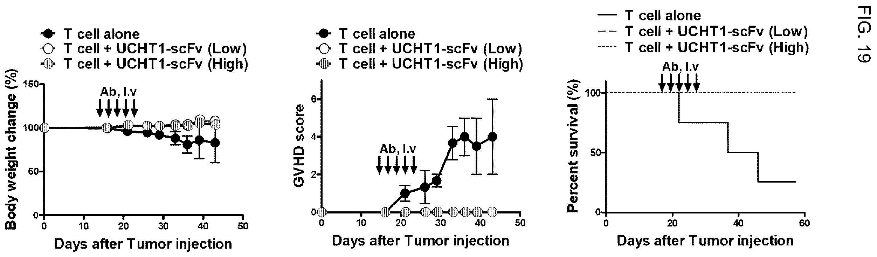

Although it is very difficult to directly demonstrate the suppressive effect of an anti-CD3 antibody on autoimmune diseases in experimental animal models, whether human T cell-mediated inflammatory diseases can be suppressed may be evaluated based on the suppressive effect on xenogeneic graft-versus-host disease induced when human T cells are administered to immunodeficient mice. In the present invention, as a result of evaluating the severity of xenogeneic graft-versus-host disease induced by human T cells in an immunodeficient mouse model, it was confirmed that, upon treatment with the monovalent anti-CD3 antibody, body weight loss, clinical symptoms, and mortality caused by graft-versus-host disease were all significantly reduced in the antibody-treated group (FIG. 19). Therefore, since the monovalent anti-CD3 antibody can markedly suppress inflammatory diseases caused by T cells, it has a very high potential to be developed as a therapeutic agent for autoimmune diseases, which are similar T cell-mediated inflammatory diseases. In addition, as a disease similar to graft-versus-host disease in the above model, the monovalent anti-CD3 antibody may also be used for the treatment of allogeneic GVHD caused by donor T cells after allogeneic hematopoietic stem cell transplant for the treatment of hematologic malignancies.

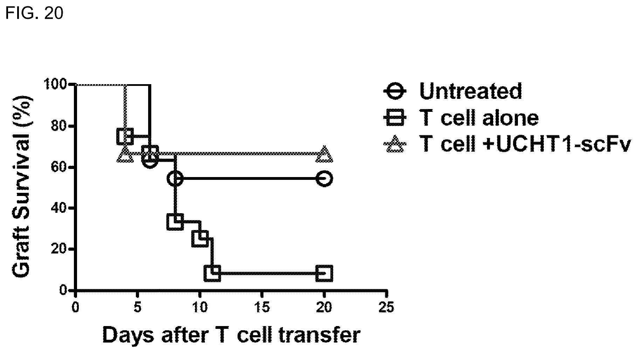

In addition, the monovalent anti-CD3 antibody or antigen-binding fragment thereof may be used as a therapeutic agent for suppressing organ transplant rejection mediated by recipient T cells following organ transplantation. In the present invention, immune rejection was induced by transplanting skin from an allogeneic strain (C57BL/6) mouse into immunodeficient NSG mice and administering activated human T cells, thereby impairing engraftment of the transplanted skin due to T cell-mediated immune rejection. In this model, in the group administered with human T cells alone, transplant rejection in which most of the transplanted skin tissue was lost was observed, whereas in the group administered with the monovalent anti-CD3 antibody in combination, it was confirmed that the skin engraftment rate was significantly improved (FIG. 20). Accordingly, the monovalent anti-CD3 antibody will be useful as an immunosuppressive agent for suppressing immune rejection during organ transplantation.

Accordingly, the monovalent anti-CD3 antibody or monovalent antigen-binding fragment thereof according to the present invention may be used as a therapeutic agent for T cell-mediated inflammatory diseases, including autoimmune diseases, graft-versus-host disease, and organ transplant rejection.

The pharmaceutical composition may further comprise an immunosuppressive agent or may be administered in combination with an immunosuppressive agent. The combination administration may mean administering the monovalent anti-CD3 antibody or monovalent antigen-binding fragment thereof and the immunosuppressive agent simultaneously, sequentially, or separately, and in any order.

In addition, another aspect of the present invention provides a pharmaceutical composition for suppressing T-cell immunity or deleting activated T cells, comprising a monovalent antibody or a monovalent antigen-binding fragment thereof that specifically binds to CD3, wherein the monovalent antibody comprises a heavy chain variable region and a light chain variable region.

In the pharmaceutical composition, the terms "antibody," "antigen-binding fragment," or "antibody or antigen-binding fragment that specifically binds to CD3" are as described above.

The monovalent antibody or monovalent antigen-binding fragment thereof that specifically binds to CD3 may induce death of activated T cells. The death may include apoptosis of T cells. In one embodiment of the present invention, unlike a full IgG form of the same antibody, only the monovalent antibody or the monovalent antigen-binding fragment thereof may induce death of T cells. In addition, the monovalent antibody or monovalent antigen-binding fragment thereof that specifically binds to CD3 may not exhibit activity of inducing death in non-activated T cells, specifically naïve T cells. More specifically, the monovalent antibody or monovalent antigen-binding fragment thereof that specifically binds to CD3 may induce apoptosis by upregulating dephosphorylation of NFATc2 in activated T cells. That is, apoptosis may be induced through the calcium-NFAT pathway during TCR/CD3 signaling in T cells. Accordingly, the monovalent antibody or monovalent antigen-binding fragment thereof may be used for suppressing T-cell immunity or depleting activated T cells.

Another aspect of the present invention provides a method for suppressing T-cell immunity, comprising administering a monovalent antibody or a monovalent antigen-binding fragment thereof that specifically binds to CD3, wherein the monovalent antibody comprises a heavy chain variable region and a light chain variable region.

In addition, the present invention provides a method for depleting TCR-positive CAR-T cells in a process of manufacturing CAR-T cells for allogeneic CAR T cell therapy, the method comprising:

(a) downregulating expression of a T cell receptor (TCR) in T cells;
(b) introducing a chimeric antigen receptor (CAR) into the T cells; and
(c) treating the cells obtained in steps (a) and (b) with a monovalent antibody or a monovalent antigen-binding fragment thereof that specifically binds to CD3, wherein the monovalent antibody comprises a heavy chain variable region and a light chain variable region,

wherein steps (a) and (b) are performed in any order.

According to one embodiment of the present invention, the method may further comprise activating T cells prior to performing each of steps (a) or (b). This is because an activated state of the target T cells is required in advance in order to downregulate expression of the T cell receptor (TCR) or to introduce a chimeric antigen receptor.

More specifically, steps (a) and (b) may be performed irrespective of chronological order. In detail, after expression of the T cell receptor is downregulated in T cells in step (a), the chimeric antigen receptor may be introduced into the T cells in step (b). Alternatively, after introduction of the chimeric antigen receptor in step (b), expression of the T cell receptor in the T cells may be downregulated in step (a). That is, the present invention provides a method in which steps (a) and (b) are performed in any order, followed by step (c), which comprises treating the cells with a monovalent antibody or a monovalent antigen-binding fragment thereof that specifically binds to CD3, wherein the monovalent antibody comprises a heavy chain variable region and a light chain variable region, to thereby deplete TCR-positive CAR-T cells.

According to one embodiment of the present invention, in allogeneic CAR T cell therapy, when CAR-T cells are manufactured by expressing a CAR in donor T cells and then administered to a recipient, the T cell receptor (TCR) of the donor T cells may recognize normal cells of the recipient as antigens and attack the normal cells, thereby causing a side effect in the form of graft-versus-host disease. In order to minimize such side effects, a process of depleting the TCR of donor T cells, specifically including step (a), is included in the manufacture of CAR-T cells for allogeneic CAR T cell therapy. In this case, despite experimental methods for downregulating expression of the TCR, residual TCR-positive CAR-T cells may remain, and such residual cells may induce inflammatory responses, namely graft-versus-host disease, in the recipient. Accordingly, residual TCR-positive CAR-T cells may be depleted through step (c), which comprises treating the cells with a monovalent antibody or a monovalent antigen-binding fragment thereof that specifically binds to CD3, wherein the monovalent antibody comprises a heavy chain variable region and a light chain variable region. More specifically, CAR-T cells in which residual TCR-positive CAR-T cells are effectively depleted may be manufactured through step (c) above.

The step of downregulating expression of the TCR may be performed by a genome editing technique, including a CRISPR system, specifically CRISPR/Cas9, TALEN, zinc finger nuclease, base editing, and prime editing, or may be performed by a nucleic acid selected from the group consisting of antisense RNA, antagomir RNA, siRNA, shRNA, and miRNA.

As used herein, the term "disease" may refer to a pathological condition, particularly cancer, infectious diseases, inflammatory diseases, degenerative diseases, apoptosis-related diseases, and graft rejection.

As used herein, the term "prevention" refers to the prevention of, or prophylactic treatment for, a disease or disease condition, and the term "treatment" refers to, or includes, alleviation, suppression of progression, or prevention of a disease, disorder, or condition, or one or more symptoms thereof. The term "active ingredient" or "pharmaceutically effective amount" may refer to any amount of a composition used in practicing the invention provided herein that is sufficient to alleviate, suppress progression of, or prevent a disease, disorder, or condition, or one or more symptoms thereof.

As used herein, the terms "administering," "introducing," and "transplanting" may be used interchangeably and may refer to placement of a composition according to one embodiment into a subject by a method or route that results in at least partial localization to a desired site. A composition according to one embodiment may be administered by any suitable route that delivers at least a portion of the cells or cellular components of the composition to a desired location in a living subject. The survival period of the cells after administration to the subject may range from as short as several hours, for example within 24 hours, to several days, or as long as several years.

The composition according to the invention may further comprise a pharmaceutically acceptable carrier. As a pharmaceutically acceptable carrier, a binder, a glidant, a disintegrant, an excipient, a solubilizer, a dispersant, a stabilizer, a suspending agent, a pigment, a flavor, and the like may be used for oral administration. A buffer, a preserving agent, a pain-relieving agent, a solubilizer, an isotonic agent, a stabilizer, and the like may be used in admixture for injections. A base, an excipient, a lubricant, a preserving agent, and the like may be used for topical administration. The pharmaceutical composition according to the present invention may be formulated in various ways by mixing with pharmaceutically acceptable carriers as described above. For example, for oral administration, the pharmaceutical composition may be formulated in the form of tablets, troches, capsules, elixirs, suspensions, syrups, wafers, or the like. For injections, the pharmaceutical composition may be formulated in the form of unit dosage ampoules or multiple dosage forms. In addition, the anticancer composition may typically include a surfactant to facilitate migration across membranes. Such surfactant may include those derived from steroids, cationic lipids such as N-[1-(2,3-dioleoyl)propyl-N,N,N-trimethylammonium chloride (DOTMA), or various compounds such as cholesterol hemisuccinate and phosphatidyl glycerol.

The pharmaceutical composition of the present invention may be administered orally or parenterally, and may be administered, for example, by intravenous injection, subcutaneous injection, intradermal injection, intramuscular injection, intraperitoneal injection, intrastemal injection, intratumoral injection, topical administration, intranasal administration, intracerebral administration, intracranial administration, intrapulmonary administration, or rectal administration, but is not limited thereto.

An appropriate dosage of the pharmaceutical composition of the present invention may vary depending on factors such as formulation method, route of administration, age, body weight, sex, pathological condition of a patient, diet, administration time, excretion rate, and responsiveness, and a person skilled in the art can readily determine and prescribe an effective dosage for desired treatment or prevention. According to a preferred embodiment of the present invention, the daily dosage of the pharmaceutical composition of the present invention is 0.0001 to 100 mg/kg. As used herein, the term "pharmaceutically effective amount" refers to an amount sufficient to prevent or treat the diseases described above.

Another aspect of the present invention provides a method for preventing or treating a disease (e.g., a T cell-mediated autoimmune disease, graft-versus-host disease, or organ transplant rejection), comprising administering, to a subject in need thereof, a monovalent antibody or a monovalent antigen-binding fragment thereof that specifically binds to CD3, wherein the monovalent antibody comprises a heavy chain variable region and a light chain variable region, or a composition comprising the same. Another aspect of the present invention provides an antibody or an antigen-binding fragment thereof comprising:

a light chain variable region comprising LCDR1 of SEQ ID NO: 23, LCDR2 of SEQ ID NO: 24, and LCDR3 of SEQ ID NO: 25, and a heavy chain variable region comprising HCDR1 of SEQ ID NO: 26, HCDR2 of SEQ ID NO: 27, and HCDR3 of SEQ ID NO: 28; or

a light chain variable region comprising LCDR1 of SEQ ID NO: 32, LCDR2 of SEQ ID NO: 33, and LCDR3 of SEQ ID NO: 34, and a heavy chain variable region comprising HCDR1 of SEQ ID NO: 35, HCDR2 of SEQ ID NO: 36, and HCDR3 of SEQ ID NO: 37.

The terms "antibody" and "antigen-binding fragment" are as described above.

The antibody or antigen-binding fragment thereof may be an antibody or antigen-binding fragment that specifically binds to CD3.

The antibody or antigen-binding fragment thereof may comprise a light chain variable region comprising the amino acid sequence of SEQ ID NO: 29 or 38 and/or a heavy chain variable region comprising the amino acid sequence of SEQ ID NO: 30 or 39.

Specifically, the antibody or antigen-binding fragment thereof may comprise a light chain variable region comprising the amino acid sequence of SEQ ID NO: 29 and/or a heavy chain variable region comprising the amino acid sequence of SEQ ID NO: 30. In addition, the antibody or antigen-binding fragment thereof may comprise a light chain variable region comprising the amino acid sequence of SEQ ID NO: 38 and/or a heavy chain variable region comprising the amino acid sequence of SEQ ID NO: 39.

Another aspect of the present invention provides a polynucleotide encoding the antibody or antigen-binding fragment thereof described above.

The polynucleotide encoding the antibody of the present invention may be readily isolated and sequenced using conventional procedures. For example, oligonucleotide primers designed to specifically amplify the heavy chain- and light chain-coding regions from phage template DNA may be used. Once the polynucleotide is isolated, it may be inserted into an expression vector, and the expression vector may then be introduced into an appropriate host cell, thereby producing a desired monoclonal antibody from the transformed host cell (i.e., transformant).

In addition, another aspect of the present invention provides an expression vector comprising the polynucleotide described above.

The expression vector may be an adenoviral vector, a retroviral vector, a lentiviral vector, or an adeno-associated virus vector, and in one embodiment of the present invention, the expression vector may be a retroviral vector. The expression vector may be constructed by a person skilled in the art such that the anti-CD3 monovalent antibody according to the present invention can be expressed and secreted. The expression vector may further comprise a signal sequence or a leader sequence. In one embodiment of the present invention, the leader sequence may comprise the sequence METDTLLLWVLLLWVPGSTGDV or MERHWIFLLLLSVTAGVHS, but is not limited thereto. In addition, the expression vector may further comprise a restriction enzyme cleavage site sequence. Specifically, the restriction enzyme cleavage site sequence may comprise the sequence AQAA, GQAGQ, or KL, but is not limited thereto.

In addition, the present invention provides a cell comprising the polynucleotide or an expression vector comprising the polynucleotide.

The cell may be transduced with a virus comprising a polynucleotide capable of expressing the antibody or antigen-binding fragment thereof that specifically binds to CD3 according to the present invention. The transduced cell may secrete the antibody or antigen-binding fragment thereof that specifically binds to CD3.

The virus may comprise, in its genome, a nucleic acid molecule encoding the antibody or antigen-binding fragment thereof that specifically binds to CD3 according to the present invention. Accordingly, a cell transduced with the virus may express an anti-CD3 antibody or a fragment thereof on its surface. The virus may be an adenovirus, a retrovirus, a lentivirus, or an adeno-associated virus. In one embodiment of the present invention, the virus may be a retrovirus. The virus may be obtained by transfecting a cell with the expression vector as described above together with an expression vector comprising a nucleic acid molecule encoding a viral envelope protein. The transfection may be performed using conventional methods. Envelope proteins usable for transfection may include VSV-G, ecotropic envelope, Mokola, Rabies, MLV-Ampho, MLV-10A1, LCMV-WE, LCMV-Arm53b envelope, feline endogenous gamma retrovirus (RD114) envelope and variants thereof, gibbon ape leukemia virus (GALV) envelope and variants thereof, MLV 4070A envelope, or gp120/gp41 (Mol. Ther. Methods Clin. Dev., 2016(3):16017; J. Virol. Methods, 2004:122-131; Molecular Therapy-Methods & Clinical Development 2016(3):16017).

## Effect of Invention

According to one aspect of the present invention, the monovalent anti-CD3 antibody or monovalent antigen-binding fragment thereof can selectively deplete activated T cells while relatively sparing non-activated T cells, and thus may be usefully employed as a T-cell depleting agent or a T-cell immunosuppressive agent. For example, it may be used for preventing or treating T cell-mediated autoimmune diseases, graft-versus-host disease, or organ transplant rejection. In addition, since the monovalent anti-CD3 antibody or monovalent antigen-binding fragment thereof can completely deplete residual donor CD3-positive T cells that may problematic in allogeneic CAR T cell therapy, which has attracted attention as an anticancer T cell therapy, it may be effectively used to prevent or ameliorate side effects of graft-versus-host disease (GVHD) caused by administration of allogeneic CAR T cells.

## Brief Description of Drawings

FIG. 1 is a graph showing the cell death effects of anti-CD3 OKT3 IgG, Fab, and scFv antibodies on activated T cells.
FIG. 2 is a graph showing the apoptosis-inducing effects of anti-CD3 OKT3 IgG, Fab, and scFv antibodies on activated T cells.
FIG. 3 is a graph showing the cell death effects of anti-CD3 UCHT1 IgG and scFv antibodies and anti-CD3 1-4-2 and 1-4-7 scFv antibodies on activated T cells.
FIG. 4 is a graph showing the cell death effects of anti-CD3 OKT3 IgG, Fab, and scFv antibodies on non-activated T cells.
FIG. 5 is an image showing analysis of the apoptosis-inducing mechanism of anti-CD3 scFv by Western blotting.
FIG. 6 is an image showing analysis of the apoptosis-inducing mechanism of anti-CD3 scFv using signaling inhibitors and flow cytometry (Veh: vehicle, CsA: cyclosporin A, Dasa: dasatinib).
FIG. 7 is a graph showing analysis of residual TCR-positive cells after TCR depletion by CRISPR/Cas9 for manufacturing allogeneic CAR T cells.
FIG. 8 is a graph showing analysis of residual TCR-positive cells after TCR depletion by CRISPR/Cas9 and MACS for manufacturing allogeneic CAR T cells.
FIG. 9 is a graph showing analysis of residual TCR-positive cells after TCR depletion by CRISPR/Cas9 and anti-CD3 OKT3 scFv for manufacturing allogeneic CAR T cells.
FIG. 10 is a graph showing analysis of allogeneic CAR T cells manufactured by CRISPR/Cas9 and anti-CD3 OKT3 scFv (Ab: CD3 OKT3 scFv).
FIG. 11 is a graph showing analysis of tumor-killing activity and cytokine secretion of allogeneic CAR T cells manufactured by CRISPR/Cas9 and anti-CD3 OKT3 scFv (Del Ab T cells: TCR-depleted + anti-CD3 OKT3 scFv T cells; Conv CAR-T: conventional CAR-T; Del Ab CAR-T: TCR-depleted CAR-T + anti-CD3 OKT3 scFv).
FIG. 12 is an image showing anticancer effects of TCR-depleted CAR T cells and/or anti-CD3 OKT3 scFv (Conv CAR-T: conventional CAR-T; Del CAR-T: TCR-depleted CAR-T; Del Ab CAR-T: TCR-depleted CAR-T + anti-CD3 OKT3 scFv).
FIG. 13 is a graph showing prevention or improvement of graft-versus-host disease-related side effects and mouse survival rates by TCR-depleted CAR T cells and/or anti-CD3 OKT3 scFv; Conv CAR-T: conventional CAR-T; Del CAR-T: TCR-depleted CAR-T; Del Ab CAR-T: TCR-depleted CAR-T + anti-CD3 OKT3 scFv.
FIG. 14 is a graph showing a reduction in the total number of T cells induced by treatment with anti-CD3 OKT3 scFv.
FIG. 15 is a graph showing changes in the numbers of CD4 and CD8 T-cell subsets by anti-CD3 OKT3 scFv treatment through flow cytometry (EF/EM: effector T cells/effector memory T cells; N/SCM: naïve/stem cell memory T cells; CM: central memory T cells).
FIG. 16 is a graph showing changes in the distribution of CD4 and CD8 T-cell subsets by anti-CD3 OKT3 scFv treatment (EF/EM: effector T cells/effector memory T cells; N/SCM: naïve/stem cell memory T cells; CM: central memory T cells).
FIGS. 17 and 18 show results of depletion of human T cells after *in vivo* administration of a monovalent anti-CD3 antibody (UCHT1-scFv).
FIG. 19 shows results of evaluating body weight loss, clinical symptoms of graft-versus-host disease, and mortality when monovalent anti-CD3 antibody (UCHT1-scFv) was administered after inducing xenogeneic graft-versus-host disease by human T cells in an immunodeficient mouse model.
FIG. 20 shows results of observing whether graft rejection occurs due to detachment of skin tissue induced by human T cells when monovalent anti-CD3 antibody (UCHT1-scFv) is administered in an immunodeficient mouse model.

**Mode for Carrying Out the Invention**

Hereinafter, preferred examples are presented to facilitate understanding of the present invention. However, the following examples are provided merely for ease of understanding of the present invention, and the scope of the present invention is not limited by the examples described below. The examples may be subjected to various modifications, and the present invention is not limited to the examples disclosed herein but may be implemented in various forms.

**Preparation Example 1. Preparation of Activated T Cells**

T cells receive activation and inactivation signals through signaling via the T cell receptor (TCR)-CD3 complex on the cell surface. The TCR is composed of alpha and beta chains, and the CD3 complex is composed of gamma, delta, epsilon, and zeta chains. Among these, antibodies against the CD3 epsilon subunit are the best known, and when anti-CD3 epsilon antibodies (hereinafter, anti-CD3 antibodies) are applied to T cells in a form coated on a solid phase (for example, antibodies coated on cell culture plates, so-called plate-bound antibodies), multiple antibodies simultaneously cross-link the TCR-CD3 complex, thereby transmitting TCR signals into the cell interior. TCR signals contain both activation and inactivation signals simultaneously. When an antibody against CD28, an activation receptor on the T cell surface, is added to provide CD28 signals together, the activation signal predominates and T cells become activated. Accordingly, activated T cells were produced by stimulating normal human peripheral blood T cells in vitro with plate-bound anti-CD3 antibody and anti-CD28 antibody.

Specifically, peripheral blood mononuclear cells (PBMCs), which are enriched in T cells, were isolated from peripheral blood of healthy donors using Ficoll centrifugation, a cell separation method based on differences in cell density. The isolated PBMCs were cultured for 5 days in the presence of plate-bound anti-CD3 antibody (10 μg/mL, clone OKT3), anti-CD28 antibody (2 μg/mL, clone CD28.2), and recombinant human IL-2 (hIL-2; 200 U/mL, Proleukin) to induce activation. During this period, only T cells survived and proliferated, and thus, after 5 days, most of the surviving cells were activated T cells. Thereafter, the cells were washed and further cultured for 3 days in a culture medium containing hIL-2 (200 U/mL), and the resulting cells were used as activated T cells.

**Example 1. Selective Depletion Ability of Monovalent Anti-CD3 Antibody Against Activated T Cells**

**1.1 Selective Depletion Ability of OKT3 Against Activated T Cells**

Anti-human CD3 OKT3 IgG antibody was purchased as a commercial antibody. In order to evaluate the T-cell depleting activity of a monovalent soluble anti-CD3 antibody, the well-known anti-human CD3 antibody OKT3 clone was isolated and purified from human HEK293F cells in the form of monovalent antibodies, Fab and scFv.

Specifically, anti-human CD3 OKT3 Fab antibody was produced by cloning the VH-CH1 domain and VL-CL domain of each clone into expression vectors (pCEP4), then transfecting the two plasmids into HEK293F cells, and purifying the antibody secreted into the culture medium by affinity purification via kappa-select resin. Specifically, anti-human CD3 OKT3 scFv antibody was produced by cloning the scFv portion of each clone connected to Ck (kappa light chain constant domain) (scFv-Ck) into pCEP4, transfecting this plasmid into HEK293F cells, and purifying the scFv-Ck antibody secreted into the culture medium by affinity purification via kappa-select resin.

Tables 1 2 below show the sequences of OKT3 scFv and OKT3 Fab, respectively, as well as the sequences used for their isolation and purification.

Table 1.

| OKT3-scFv-Ck | |
|---|---|
| leader sequence | METDTLLLWVLLLWVPGSTGDV |
| restriction enzyme site | AQAA |
| OKT3-VL | QIVLTQSPAIMSASPGEKVTMTCSASSSVSYMNWYQQKSGTSPKRWIYDTSKLASGVPAHFRGSGSGTSYSLTISGMEAEDAATYYCQQWSSNPFTFGSGTKLEINR |
| (G4S)3 linker | GGGGSGGGGSGGGGS |

(continued)

| OKT3-scFv-Ck | |
|---|---|
| OKT3-VH | QVQLQQSGAELARPGASVKMSCKASGYTFT<u>RYTMH</u>WVKQRPGQGLEWIG<u>YINPSRGYTNYNQKFKD</u>KATLTTDKSSSTAYMQLSSLTSEDSAVYYCAR<u>YYDDHYCLDY</u>WGQGTTLTVSS |
| restriction enzyme site | GQAGQ |
| (G4S)3 linker | GGGGSGGGGSGGGGS |
| human C kappa | RTVAAPSVFIFPPSDEQLKSGTASVVCLLNNFYPREAKVQWKVDNALQSGNSQESVTEQDSKDSTYSLSSTLTLSKADYEKHKVYACEVTHQGLSLPVTKSFNRGES |
| linker | GGA |
| HA tag | YPYDVPDYAS |

Table 2.

| OKT3-Fab-Heavy chain | |
|---|---|
| leader sequence | MERHWIFLLLLSVTAGVHS |
| OKT3-VH | QVQLQQSGAELARPGASVKMSCKASGYTFT<u>RYTMH</u>WVKQRPGQGLEWIG<u>YINPSRGYTNYNQKFKD</u>KATLTTDKSSSTAYMQLSSLTSEDSAVYYCAR<u>YYDDHYCLDY</u>WGQGTTLTVSS |
| mouse IgG1 heavy chain CH1 region | AKTTPPSVYPLAPGSAAQTNSMVTLGCLVKGYFPEPVTVTWNSGSLSSGVHTFPAVLQSDLYTLSSSVTVPSSPRPSETVTCNVAHPASSTKVDKKIVPRDC |
| **OKT3-Fab-Light chain** | |
| leader sequence | MDFQVQIFSFLLISASVIISRG |
| OKT3-VL | QIVLTQSPAIMSASPGEKVTMTC<u>SASSSVSYMN</u>WYQQKSGTSPKRWIY<u>DTSKLAS</u>GVPAHFRGSGSGTSYSLTISGMEAEDAATYYC<u>QQWSSNPFT</u>FGSGTKLEIN |
| mouse IgG1 | RADAAPTVSIFPPSSEQLTSGGASVVCFLNNFYPKDINVKWKID |
| light chain constant region | GSERQNGVLNSWTDQDSKDSTYSMSSTLTLTKDEYERHNSYTCEATHKTSTSPIVKSFNRNEC |

Each form of purified antibodies were treated to human T cells activated for 8 days at the same molar concentration (12.5 pmol antibody/$5 \times 10^5$ T cells/mL), and after 24 hours, T cell viability and apoptosis were analyzed by flow cytometry.

Specifically, activated T cells were treated with each form of the OKT3 antibody (Fab or scFv) and cultured for 24 hours, after which T-cell viability was analyzed by 7-AAD staining and flow cytometry. 7-AAD is a fluorescent DNA-binding compound that cannot penetrate intact cell membranes and therefore does not bind DNA in viable cells; however, when cells undergo death and the cell membrane is disrupted, 7-AAD enters the cells, binds to DNA, and emits fluorescence.

Accordingly, 7-AAD-negative cells were classified as live cells, whereas 7-AAD-positive cells were classified as dead or dying cells.

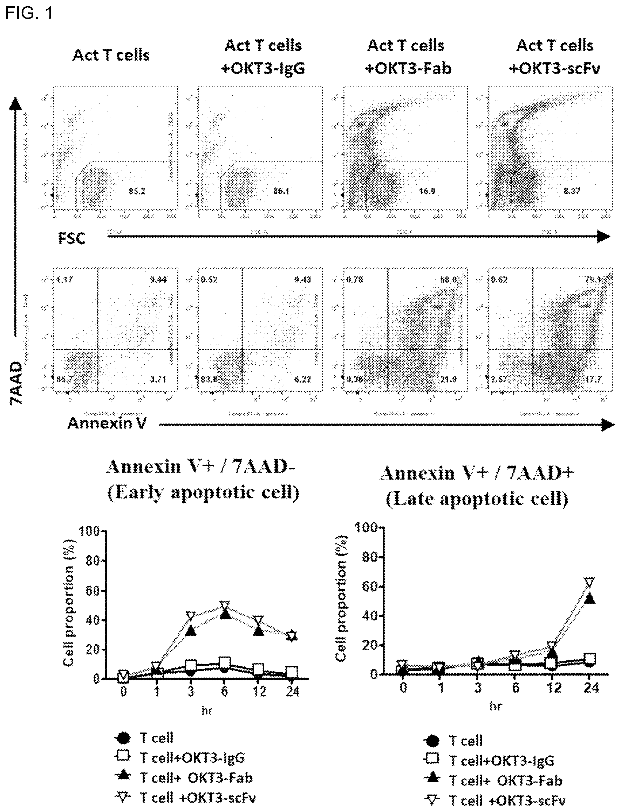

As shown in FIG. 1, the soluble OKT3 antibody in a full IgG form did not affect T-cell viability, whereas the monovalent antibody forms, OKT3-Fab and OKT3-scFv, were confirmed to significantly reduce T-cell viability, as indicated by a marked decrease in the proportion of 7-AAD-negative cells.

### 1.2 Mechanism of Activated T Cell Death - Apoptosis

To confirm whether the death of activated T cells by monovalent antibody forms OKT3-Fab and OKT3-scFv antibodies was due to apoptosis, Annexin V, a marker of apoptotic cell death, and 7AAD staining were performed simultaneously, and the results are shown in FIG. 1. In early apoptotic cells, the cell membrane is stained with Annexin V, but cell membrane disruption has not yet progressed, so they display an Annexin V(+) 7AAD(-) form without 7AAD staining; however, late apoptotic cells are stained with 7AAD as well, displaying an Annexin V(+) 7AAD(+) form.

In order to determine whether death of activated T cells induced by the monovalent antibody forms OKT3-Fab and OKT3-scFv occurred via apoptosis, Annexin V, a marker of apoptotic cell death, and 7-AAD staining were performed simultaneously, and the results are shown in FIG. 1. In early apoptotic cells, the cell membrane is stained with Annexin V, but membrane disruption has not yet occurred, and thus 7-AAD is not stained, resulting in an Annexin V-positive/7-AAD-negative [Annexin V(+)/7-AAD(-)] profile. In contrast, late apoptotic cells are stained with 7-AAD, resulting in an Annexin V-positive/7-AAD-positive [Annexin V(+)/7-AAD(+)] profile.

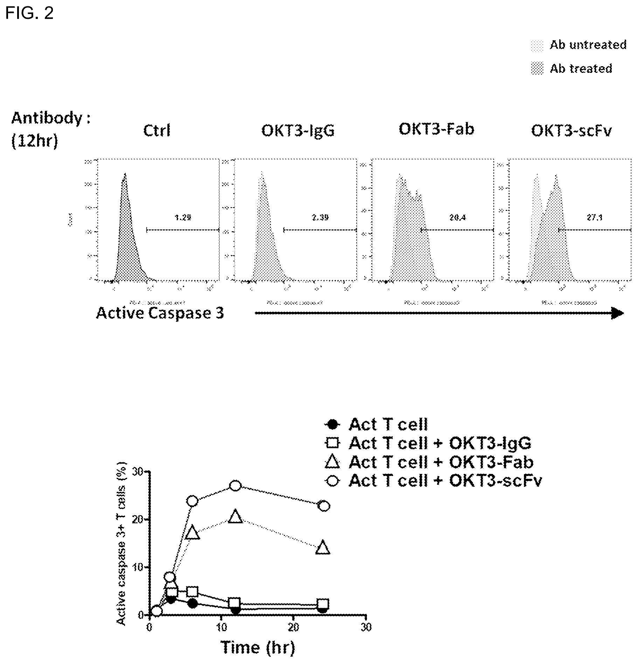

In addition, to further verify that apoptosis of activated T cells was induced by the monovalent OKT3 antibodies, activation of caspase-3, an enzyme mediating apoptosis, was analyzed by flow cytometry, and the results are shown in FIG. 2.

As shown in FIG. 1, analysis over time after treatment of activated T cells with each form of the OKT3 antibody revealed that, in the groups treated with OKT3-Fab or OKT3-scFv antibodies, the proportion of early apoptotic cells increased sharply starting from 3 hours, and late apoptotic cells began to appear after 6 hours, followed by a rapid increase in their proportion thereafter. In contrast, in the group treated with the OKT3 antibody in a full IgG form, there was no change in the proportion of apoptotic cells.

As shown in FIG. 2, in the groups treated with OKT3-Fab or OKT3-scFv antibodies, activated caspase-3 (cleaved caspase-3) was detected starting from 3 hours after treatment, indicating that active apoptosis occurred in these cells. However, activated caspase-3 was not detected in the group treated with the OKT3 antibody in a full IgG form.

From these results, it was confirmed that, unlike the OKT3 antibody in a full IgG form, OKT3 in Fab or scFv form induces cell death of activated T cells via an apoptosis pathway.

### 1.3 Selective Depletion Ability of Other Monovalent Anti-CD3 Antibodies Against Activated T Cells

It was examined whether the apoptosis-inducing activity of the soluble monovalent OKT3 antibody against T cells could be reproduced with other monovalent anti-CD3 antibodies. Another anti-CD3 antibody, UCHT1, was evaluated, wherein the IgG form of the UCHT1 antibody was purchased as a commercial antibody, and an scFv form thereof was prepared in the same manner as described in Section 1.1 above. Thereafter, experiments were conducted to determine whether the UCHT1 scFv induced death of activated T cells.

Table 3 below shows the sequence of UCHT1 scFv and the sequences used for its isolation and purification.

Table 3.

| UCHT1-scFv-Ck | |
|---|---|
| leader sequence | METDTLLLWVLLLWVPGSTGDV |
| restriction enzyme site | AQAA |
| UCHT1-VL | DIQMTQTTSSLSASLGDRVTISC<u>RASQDIRNYLN</u>WYQQKPDGT VKLLIY<u>YTSRLHS</u>GVPSKFSGSGSGTDYSLTISNLEQEDIATYFC <u>QQGNTLPWT</u>FAGGTKLEIKRA |
| (G4S)3 linker | GGGGSGGGGSGGGGS |

(continued)

| UCHT1-scFv-Ck | |
|---|---|
| UCHT1-VH | EVQLQQSGPELVKPGASMKISCKAS<u>GYSFTGYTMN</u>WVKQSHG KNLEWMG<u>LINPYKGVST</u>YNQKFKDKATLTVDKSSSTAYMELLSL TSEDSAVYYCAR<u>SGYYGDSDWYFDV</u>WGAGTTVTVSS |
| restriction enzyme site | GQAGQ |
| (G4S)3 linker | GGGGSGGGGSGGGGS |
| human C kappa | RTVAAPSVFIFPPSDEQLKSGTASVVCLLNNFYPREAKVQWKV DNALQSGNSQESVTEQDSKDSTYSLSSTLTLSKADYEKHKVYA CEVTHQGLSLPVTKSFNRGES |
| linker | GGA |
| HA tag | YPYDVPDYAS |

In addition, in order to verify the general applicability of apoptosis-mediated cell death induced by scFv-type anti-CD3 antibodies, novel anti-CD3 epsilon antibodies were screened. Specifically, human CD3 gamma/epsilon and delta/epsilon extracellular heterodimer proteins were mixed with adjuvant and immunized into 10 chickens each, four times total at 2-week intervals. Then, the spleen, bone marrow, and bursa of Fabricius were collected from the immunized chickens, and a chicken immune antibody library was constructed by performing total RNA isolation, cDNA synthesis, variable light chain & heavy chain gene PCR, and cloning into pComb3XSS, a phage display vector.

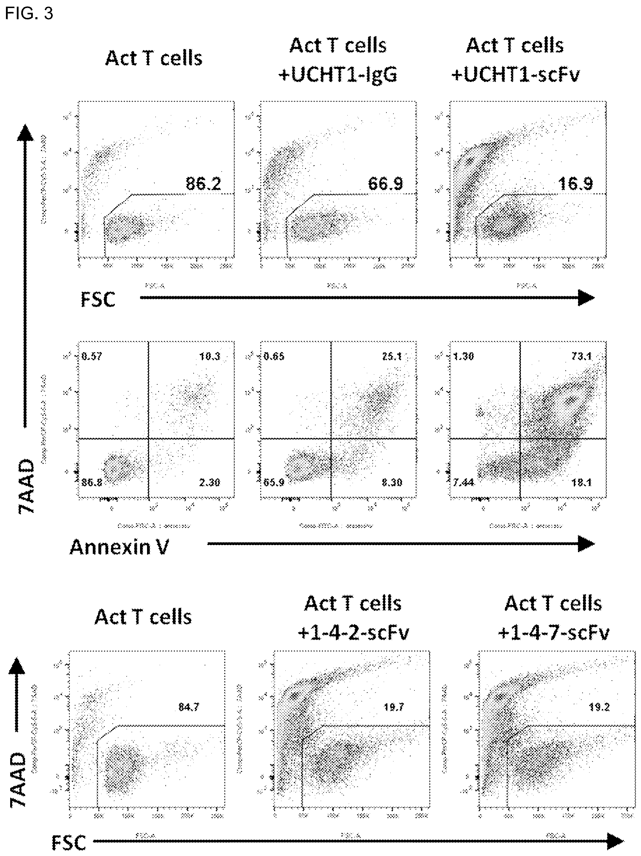

Subsequently, biopanning was performed to screen antibody clones that specifically bind to human CD3 protein. Positive antibody clones were isolated and purified in an scFv format, and two antibodies (clones 1-4-2 and 1-4-7) that bind to CD3(+) Jurkat cells were finally selected. Thereafter, experiments were conducted to analyze whether these antibodies induced death of activated T cells in the same manner as described in Section 1.1 above. The respective results are shown in FIG. 3.

Tables 4 and 5 below show the sequences of scFv of 1-4-2 and 1-4-7, respectively, and the sequences used for their isolation and purification.

Table 4.

| 1-4-2-scFv-Ck | |
|---|---|
| leader sequence | METDTLLLWVLLLWVPGSTGDV |
| restriction enzyme site | AQAA |
| 1-4-2-VL | LTQPSSVSANLGGTVKLTC<u>SGSRYKYG</u>WFQQKSPGSAPVTVIY <u>NNDKRPS</u>DIPSRFSGSASGSTATLTITGVQADDEAVYYC<u>GNQD</u> <u>TSGAV</u>FGVGTTLTVL |
| linker | GQSSRSSGGGGSSGGGGS |
| 1-4-2-VH | AVTLDESGGGLQTPGGTLSLVCKAS<u>GFSIGGYG</u>MGWVRQAPG KGLEWVG<u>TISRTGGTTWYGSAVKG</u>RATISRDSGQSTVRLQLNN LRAEDTGTYYCAR<u>ITAVCSTRSCSSRTYTVGQIDA</u>WGHGTEVIV SS |
| restriction enzyme site | TSGQAGQ |
| (G4S)3 linker | GGGGSGGGGSGGGGS |
| restriction enzyme site | KL |

(continued)

| 1-4-2-scFv-Ck | |
|---|---|
| human C kappa | RTVAAPSVFIFPPSDEQLKSGTASVVCLLNNFYPREAKVQWKV DNALQSGNSQESVTEQDSKDSTYSLSSTLTLSKADYEKHKVYA CEVTHQGLSLPVTKSFNRGES |
| His tag | HHHHHH |

Table 5.

| 1-4-7-scFv-Ck | |
|---|---|
| leader sequence | METDTLLLWVLLLWVPGSTGDV |
| restriction enzyme site | AQAA |
| 1-4-7-VL | LTQPSSVSANLGETVKITC<u>SGGSSYYG</u>WFQQKSPGSAPVTVIY <u>ENDKRPS</u>DIPSRFSGSKSGSTGTLTITGVQADDEAVYFC<u>GTYD SNYVGI</u>FGAGTTLTVL |
| linker | GQSLRSSGGGGSSGGGGS |
| 1-4-7-VH | AVTLDESGGGLQTPGGALSLVCKAS<u>GFTFNSYD</u>MGWVRQAPG KGLEFVA<u>DINSRSSKTAYGAAVKG</u>RATISRDNGQSTVRLQLNNL RAEDTATYYCAK<u>SASGGSNWPGAPASIDT</u>WGHGTEVIVSS |
| restriction enzyme site | TSGQAGQ |
| (G4S)3 linker | GGGGSGGGGSGGGGS |
| restriction enzyme site | KL |
| human C kappa | RTVAAPSVFIFPPSDEQLKSGTASVVCLLNNFYPREAKVQWKV DNALQSGNSQESVTEQDSKDSTYSLSSTLTLSKADYEKHKVYA CEVTHQGLSLPVTKSFNRGES |
| His tag | HHHHHH |

As shown in FIG. 3, the UCHT1 antibody in a full IgG form did not affect the viability of activated T cells, whereas the scFv form of the UCHT1 antibody markedly reduced T-cell viability. In addition, scFv forms of the two screened anti-CD3 antibody clones (1-4-2 and 1-4-7) also resulted in a significant reduction in T-cell viability when applied to activated T cells.

**1.4 Selective Depletion Ability of Anti-CD3 Against Non-Activated T Cells**

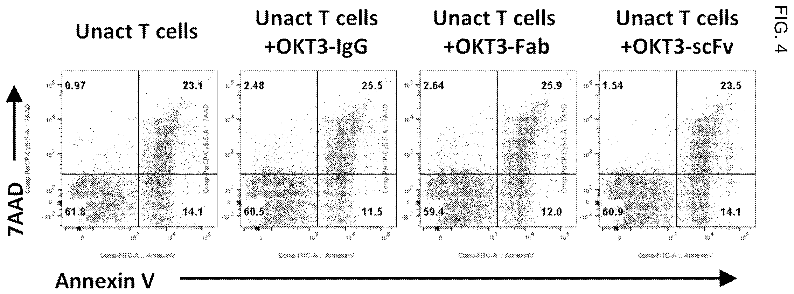

In order to examine whether the apoptosis-inducing activity of the monovalent anti-CD3 antibodies described above was also observed in non-activated T cells, peripheral blood T cells from healthy donors were treated with the scFv OKT3 antibody for 24 hours, and T-cell death was analyzed in the same manner as described in Example 1.3 above. The results are shown in FIG. 4.

As shown in FIG. 4, no apoptosis-associated cell death was observed in non-activated T cells treated with any of the OKT3 antibodies, including the full IgG, Fab, and scFv forms.

From these results, it was confirmed that the T-cell killing activity of anti-CD3 antibodies is not observed in the full IgG form, but that Fab- or scFv-form anti-CD3 antibodies exhibit selective depletion activity specifically against activated T cells. It was further confirmed that a total of four different monovalent anti-CD3 antibodies induced apoptosis of activated T cells.

**Example 2. Analysis of Apoptosis Mechanism of Monovalent Anti-CD3 Antibody**

The TCR/CD3 signaling pathways were analyzed in order to determine whether T-cell apoptosis induced by the monovalent anti-CD3 antibody is mediated through the TCR/CD3 signaling system.

TCR/CD3 signaling in T cells is known to involve activation of upstream signaling molecules such as ZAP70-LAT-PLCγ1, followed by activation of three downstream signaling pathways, namely the calcium-NFAT pathway, the PKCθ-NFκB pathway, and the Erk-AP1 pathway. Specifically, activation of the calcium-NFAT pathway can be confirmed by rapid migration of NFAT molecules on Western blotting, as NFAT is activated by dephosphorylation and dephosphorylated NFAT is detected as a band of smaller molecular size on SDS-PAGE. Activation of the PKCθ-NFκB pathway can be confirmed by degradation of IκB, which is an inhibitor of NFκB, and activation of the Erk-AP1 pathway can be confirmed by detection of phosphorylated Erk (p-Erk).

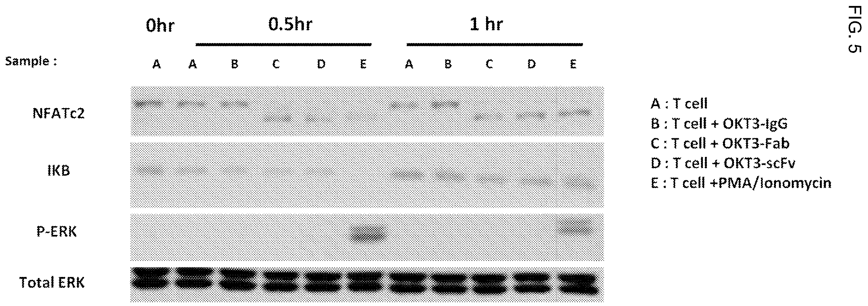

Accordingly, each form of the OKT3 antibody (OKT3-IgG, OKT3-Fab, and OKT3-scFv), or PMA/ionomycin (a positive control capable of activating all three downstream signaling pathways), was treated to activated T cells, and activation of each signaling pathway was examined by Western blotting through analysis of NFATc2, IκB, phosphorylated ERK (p-ERK), and total ERK. The results are shown in FIG. 5.

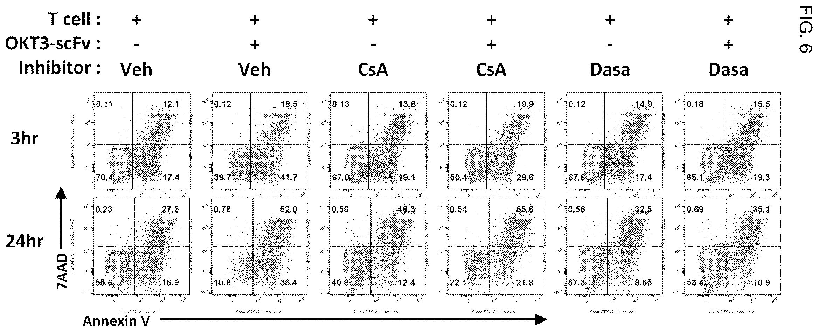

In addition, in order to verify whether activation of the TCR/CD3 signaling and its downstream NFAT pathway was responsible for apoptosis induced by the OKT3 antibody, T-cell death was examined following administration of signaling inhibitors that affect activation of upstream molecules in the TCR/CD3 signaling pathway. Specifically, activated T cells were treated with the anti-CD3 antibody in combination with cyclosporin A (CsA), a calcineurin inhibitor, at a concentration of 10 μM in 0.01% DMSO, or dasatinib, an Lck inhibitor, at a concentration of 100 nM in 0.01% DMSO. A vehicle control consisted of 0.01% DMSO alone. The results are shown in FIG. 6.

As shown in FIG. 5, when each group was compared with the positive control that induces activation of all three signaling pathways, no noticeable activation of any of the three pathways was observed in the OKT3-IgG-treated cells. In contrast, activation of NFAT was observed in cells treated with OKT3-Fab and OKT3-scFv. However, activation of the NFκB and Erk-AP1 pathways was not observed. Accordingly, it was confirmed that the monovalent OKT3 antibodies activate the NFAT pathway during TCR/CD3 signal transduction.

As shown in FIG. 6, dasatinib (Dasa) markedly suppressed apoptosis induced by OKT3-scFv, and cyclosporin A (CsA) was confirmed to partially suppress apoptosis. That is, OKT3-scFv induces apoptosis through TCR/CD3 signaling, and it was confirmed that the NFAT pathway is particularly involved in this process.

**Example 3. Depletion Activity of Anti-CD3 scFv Against Residual TCR-Positive CAR T Cells in Allogeneic CAR T Cell Therapy**

**3.1 Incompleteness of TCR Depletion by CRISPR/Cas9 and/or MACS**

Most antitumor CAR T cell therapies are currently performed in the form of autologous CAR T cell therapy, in which autologous T cells are collected from a patient's peripheral blood, genetically modified by introducing a CAR gene, and then reinfused into the same patient. In the case of autologous CAR T cell therapy, not only does the manufacturing process require a considerable amount of time, but CAR T cell production may also fail due to poor quality or impaired function of the patient's T cells.

Accordingly, allogeneic CAR T cell therapy has been explored, in which CAR T cells are pre-manufactured from peripheral blood T cells collected from healthy donors (allogeneic T cells), cryopreserved in advance, and then immediately administered to patients in need. However, in allogeneic CAR T cell therapy, the administered allogeneic CAR T cells may attack normal tissues of the recipient, thereby causing severe inflammatory side effects in the form of graft-versus-host disease (GVHD).

To prevent such GVHD, attempts have been made to manufacture CAR T cells in which the TCR/CD3 complex of T cells that induces graft-versus-host disease is depleted using genome-editing technologies such as CRISPR/Cas9. However, depletion of the TCR/CD3 complex by the CRISPR/Cas9 technique has the limitation that a population of residual TCR-positive CAR T cells may remain.

Since the TCR and CD3 are always expressed together on the cell surface in the form of a complex, if either the TCR or CD3 is not expressed, the other is likewise not expressed. Accordingly, residual TCR-positive cells can be depleted using an anti-CD3 antibody. Thus, during the process of depleting the TCR/CD3 complex by CRISPR/Cas9 for allogeneic CAR T cell therapy, the monovalent anti-CD3 antibody of the present invention was used to deplete residual TCR-positive cells.

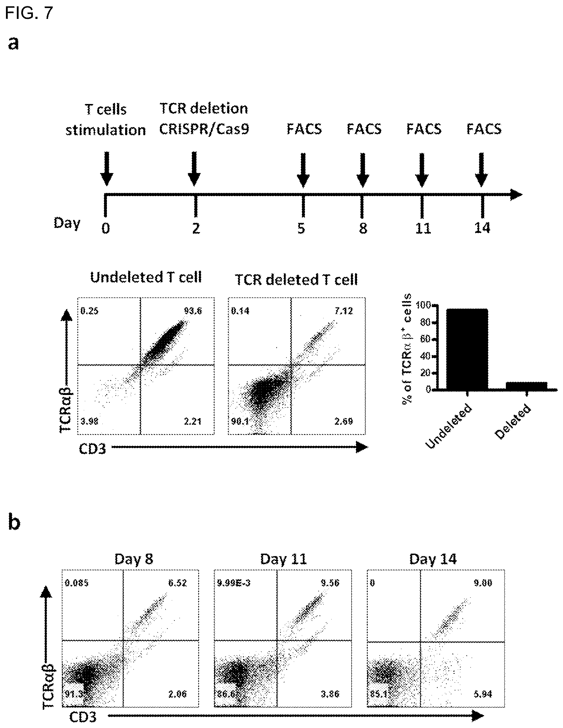

Specifically, as shown in FIG. 7, T cells were activated on day 0 of the experiment, and a gRNA/Cas9 protein complex (ribonucleoprotein; RNP) targeting the TCR was prepared using a previously reported human TCR alpha chain-specific guide RNA (gRNA) sequence (GAGAATCAAAATCGGTGAAT) (Mol. Ther. 2016 Mar;24(3):570-581). The RNP was introduced into T cells activated for 2 days by electroporation. Thereafter, the T cells were cultured for several days in a culture medium containing hIL-2 (200 U/mL), and expression of the TCR and the CD3 epsilon chain on the cell surface was analyzed by flow cytometry. Residual TCR-positive T cells were analyzed by flow cytometry on days 5, 8, 11, and 14 of the experiment, and the results are shown in FIG. 7.

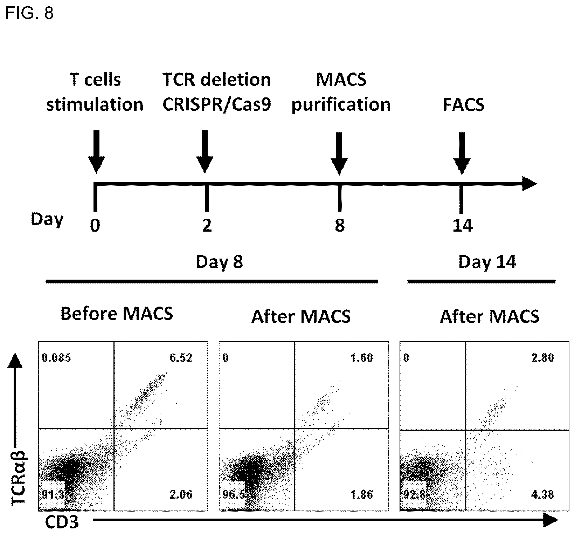

In addition, residual TCR-positive cells were examined in the same manner when an additional conventional method, magnetic-associated cell sorting (MACS), was performed to deplete residual TCR-positive cells. Specifically, after depletion of the TCR in T cells activated for 2 days using the CRISPR/Cas9 technique, the T cells were further processed 6 days later by treating them with an anti-CD3 antibody labeled with magnetic microbeads. The T cells were then passed through a magnetic column (LD column), whereby CD3-positive cells, that is, TCR-positive cells, were trapped in the column. The TCR-negative cells that passed through the column were collected and further cultured for several days in a culture medium containing hIL-2 (200 U/mL), after which residual TCR-positive T cells were analyzed by flow cytometry. The results are shown in FIG. 8.

As shown in FIG. 7, approximately 7% of TCR-positive T cells were confirmed to remain on day 5 of culture, and this residual TCR-positive fraction remained constant even when in vitro culture was continued for up to two weeks.

As shown in FIG. 8, even when the conventional MACS (magnet-associated cell sorting) purification method was used, approximately 2% of TCR-positive T cells were found to remain.

From these results, it was experimentally confirmed that complete depletion of TCR-positive T cells by the CRISPR/Cas9 technique is very difficult to achieve using the conventional MACS method alone.

### 3.2 Depletion Activity of Anti-CD3 scFv Against Residual TCR-Positive T Cells

After depletion of TCR-positive T cells using the CRISPR/Cas9 technique, it was examined whether residual TCR-positive T cells could be further depleted by adding the OKT3-scFv antibody to the cell culture medium.

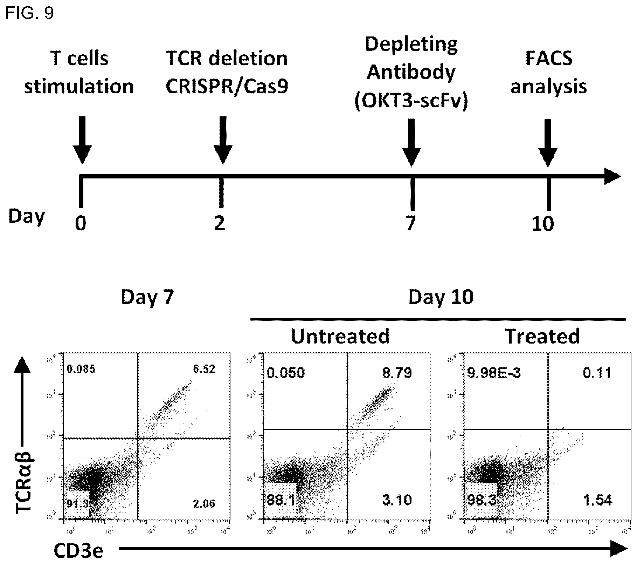

First, as shown in FIG. 9, OKT3-scFv antibody was treated to T cells on day 7 of culture, which had undergone T-cell activation and introduction of the anti-TCR gRNA/Cas9 complex, followed by additional culture for 3 days. Thereafter, the presence of residual TCR-positive T cells was analyzed, and the results are shown in FIG. 9.

As shown in FIG. 9, most of the residual TCR-positive T cells were depleted, demonstrating that treatment with OKT3-scFv more effectively depleted residual TCR-positive T cells than the conventional MACS-based purification method.

Subsequently, the depletion activity of the anti-CD3 scFv of the present invention was further evaluated by applying it to conventional anti-CD19 CAR T cells, which are most commonly used in the art, in order to assess its ability to deplete TCR-positive T cells.

Specifically, an anti-CD19 CAR cDNA was constructed in a form in which an anti-CD19 scFv, a CD8 hinge and transmembrane domain, a 4-1BB intracellular domain, and a CD3 zeta intracellular domain were linked. The anti-CD19 CAR cDNA was synthesized based on a previously disclosed sequence (U.S. Patent Application Publication No. US 2013/0287748 A1) by a commercial provider (Integrated DNA Technologies). The synthesized anti-CD19 CAR cDNA was cloned into a lentiviral vector. Thereafter, the lentiviral vector was co-transfected into a 293T cell line (ATCC) together with three packaging plasmids (pMD.2G, pMDLg/pRRE, and pRSV-rev) using Lipofectamine 3000 (Invitrogen). After 24 to 48 hours, culture supernatants containing lentiviral particles were collected, filtered through a 0.45 $\mu$m filter to remove residual cellular debris, and concentrated 100-fold by ultracentrifugation. The resulting concentrated lentiviral preparation was used for the manufacture of CAR T cells.

After activation of T cells within peripheral blood mononuclear cells for 2 days, the T cells were cultured together with the concentrated lentivirus for an additional 2 days to transduce the CAR gene into the T cells, thereby generating anti-CD19 CAR T cells. Thereafter, the cells were washed to remove residual virus in the culture medium, and proliferation of the CAR T cells was induced by adding a culture medium containing hIL-2 (200 U/mL) twice at 3-day intervals.

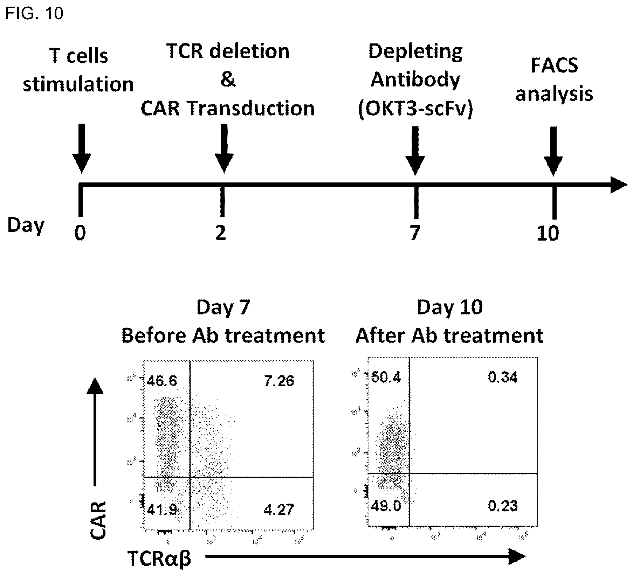

Seven days after initiation of T-cell culture, OKT3-scFv antibody (12.5 pmol/1 $\times$ 10$^5$ cells) was added to the culture medium, followed by additional culture for 3 days. On day 10 from initiation of culture, residual TCR-positive T cells were analyzed by flow cytometry, and the results are shown in FIG. 10.

As shown in FIG. 10, although more than 10% of TCR-positive T cells were detected in CAR T cells manufactured through TCR depletion and introduction of the anti-CD19 CAR gene on day 7 of culture, additional culture in the presence of OKT3-scFv for 3 days resulted in depletion of most of the residual TCR-positive CAR T cells.

In addition, in order to examine whether treatment of conventional anti-CD19 CAR T cells with the anti-CD3 scFv of the present invention affects functional properties of the CAR T cells, such as tumor-killing activity, CAR T cells were co-cultured with CD19-positive Raji cells, and cytokine secretion and tumor-killing activity were evaluated.

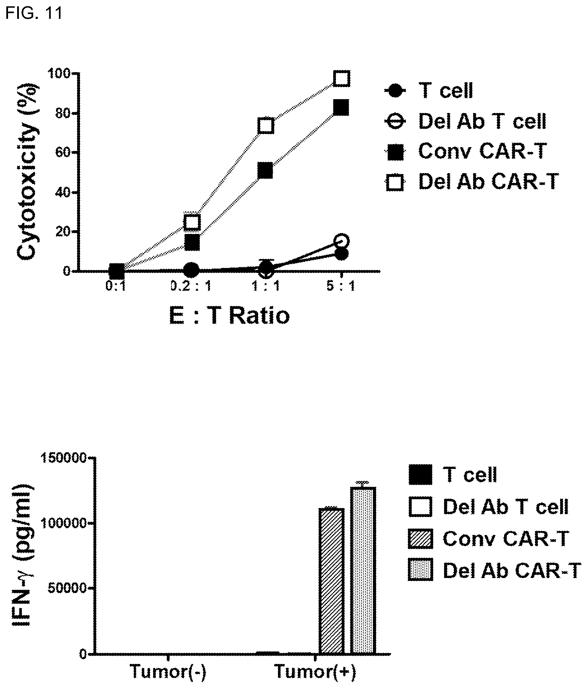

Specifically, Raji-Luc cells (3 $\times$ 10$^4$), which are CD19-positive Raji cells overexpressing luciferase, were co-cultured with CAR T cells or T cells for 18 hours. Thereafter, luciferin (6 mg/mL) was added to the culture medium, and luminescence emitted from surviving Raji-Luc cells was measured using a luminometer to determine cell viability, from which cytotoxic activity was calculated (FIG. 11). The specific equations are as follows:

[Equation 1]

Viability of Raji-Luc cells (%)

= [(luminescence of experimental group) − (background luminescence)]

/ [(luminescence of Raji-Luc cells alone) − (background luminescence)] × 100

[Equation 2]

Cytotoxic activity (% cytotoxicity) = 100 − cell viability

In addition, cytokine secretion activity was evaluated by measuring IFN-γ secreted upon co-culture of antibody-treated CAR T cells or T cells with CD19-positive tumor cells. Specifically, after co-culturing $1.5 \times 10^5$ CD19-positive Raji cells with $3 \times 10^4$ CAR T cells for 24 hours, culture supernatants were collected, and the amount of human IFN-γ in the supernatants was measured using a human IFN-γ ELISA kit (R&D Systems) according to the manufacturer's instructions (FIG. 11).

As shown in FIG. 11, both tumor-killing activity and cytokine secretion activity of the CAR T cells were maintained at levels comparable to those of CAR T cells not treated with the antibody.

From these results, it was confirmed that treatment of residual TCR-positive CAR T cells with the monovalent anti-CD3 antibody during the TCR depletion process in allogeneic CAR T cell manufacturing more effectively depletes residual TCR-positive CAR T cells, thereby providing the potential to alleviate or improve side effects such as graft-versus-host disease.

### 3.3 Prevention of Graft-Versus-Host Disease by Depletion of Residual TCR-Positive CAR T Cells Using Anti-CD3 scFv (*in vivo*)

In order to examine whether graft-versus-host disease induced by residual TCR-positive CAR T cells generated during the manufacturing process of allogeneic CAR T cells can be prevented by treatment with the monovalent anti-CD3 antibody (OKT3 scFv) of the present invention, in vivo experiments were performed.

Although it is difficult to directly model graft-versus-host disease caused by allogeneic CAR T cells in mice, administration of human CAR T cells into immunodeficient mice allows modeling of the disease by inducing recognition of murine major histocompatibility complex (MHC) molecules on normal mouse tissues by the TCRs of human CAR T cells, thereby causing attack of mouse tissues. This model mimics allogeneic graft-versus-host disease induced by donor CAR T cells recognizing recipient MHC molecules and is referred to as xenogeneic graft-versus-host disease (xeno-GVHD).

That is, when human CAR T cells expressing TCRs are administered into immunodeficient mice bearing human tumors, antitumor efficacy of the CAR T cells can be observed in vivo during the initial period of approximately one month. Thereafter, adverse effects of xenogeneic graft-versus-host disease, in which human CAR T cells attack normal mouse tissues, can be observed.

Specifically, immunodeficient NSG mice were subjected to total body irradiation at a dose of 2.5 Gy, followed by intravenous injection of $5 \times 10^{\wedge}5$ CD19-positive Raji-Luc cells. Three days after Raji cell injection, the mice were intravenously administered with $5 \times 10^{\wedge}6$ cells per mouse of one of the following: anti-CD19 CAR T cells generated from human T cells, anti-CD19 CAR T cells in which the TCR was depleted, or anti-CD19 CAR T cells in which residual TCR-positive cells were further depleted by treatment with the OKT3-scFv antibody.

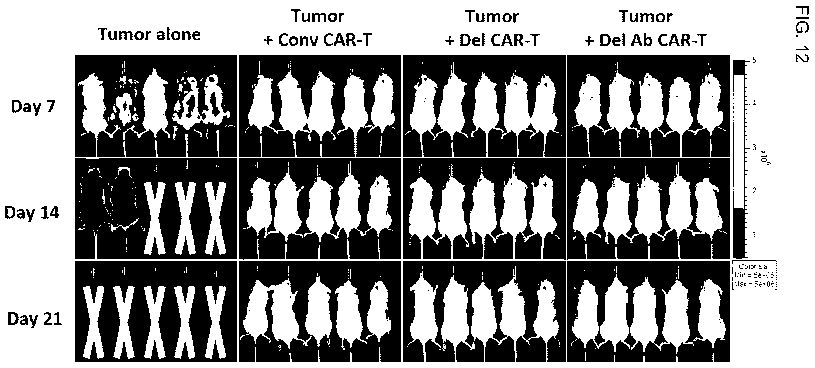

Tumor burden was monitored at weekly intervals after tumor cell injection using an in vivo imaging system (IVIS) by measuring tumor-associated bioluminescence. For imaging, each mouse was intraperitoneally injected with 2 mg of luciferin dissolved in 100 μL of saline, and tumor images were acquired using IVIS 10 minutes after luciferin injection. The results are shown in FIG. 12.

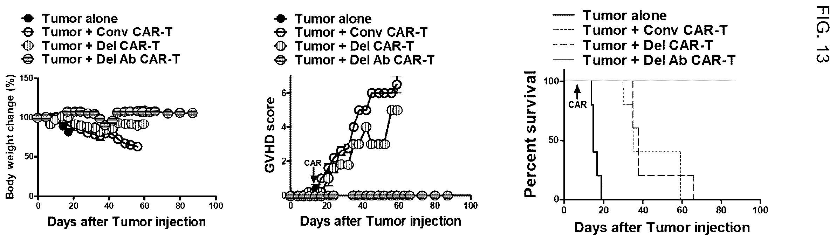

As shown in FIG. 12, longitudinal measurement of tumor growth by bioluminescence imaging demonstrated that all mice in the tumor-only control group died within 2-3 weeks. In contrast, all groups treated with the three types of CAR T cells exhibited minimal tumor growth and 100% survival up to 3 weeks, indicating that antitumor efficacy of the CAR T cells was well maintained in all treatment groups. However, at 10 weeks after tumor inoculation, all mice in the groups receiving conventional anti-CD19 CAR T cells or TCR-depleted anti-CD19 CAR T cells that still contained residual TCR-positive cells died due to graft-versus-host disease. In contrast, only the group of mice administered with anti-CD19 CAR T cells in which residual TCR-positive cells were depleted by treatment with the OKT3-scFv antibody survived without exhibiting graft-versus-host disease-related adverse effects (FIG. 13).

In addition, the severity of graft-versus-host disease was compared among groups of mice administered with anti-CD19 CAR T cells generated from human T cells, anti-CD19 CAR T cells in which the TCR was depleted but residual TCR-positive cells remained, or anti-CD19 CAR T cells in which residual TCR-positive cells were depleted by treatment with the OKT3-scFv antibody.

The severity of graft-versus-host disease was assessed based on body weight and graft-versus-host disease (GVHD) scores. Body weight and GVHD scores were measured twice per week. The GVHD score was calculated as the sum of scores (0 to 3 points each) assigned based on five clinical parameters, including skin condition, fur condition, posture, activity level, and inflammatory eye disease. Measurements were performed for up to 80 days after tumor inoculation, and the results are shown in FIG. 13.

As shown in FIG. 13, in the group administered with conventional CAR T cells in which the TCR was not depleted (Conv CAR-T), body weight loss and clinical signs of graft-versus-host disease were observed starting from two weeks after tumor inoculation, and all animals died from graft-versus-host disease by three weeks. In the group administered with CAR T cells in which the TCR was depleted but residual TCR-positive cells remained (Del CAR-T), body weight loss and the severity of graft-versus-host disease were partially reduced compared to the Conv CAR-T group; however, all animals nevertheless died from graft-versus-host disease at 8-9 weeks. This result indicates that residual TCR-positive CAR T cells induce severe graft-versus-host disease-related adverse effects.

In contrast, in the group administered with CAR T cells in which residual TCR-positive cells were completely depleted by treatment with the anti-CD3 scFv antibody (Del Ab CAR-T), all animals exhibited long-term survival without body weight loss or graft-versus-host disease symptoms, while maintaining potent antitumor efficacy without adverse effects.

From these results, it was confirmed that efficient and complete depletion of residual TCR-positive cells through treatment with a monovalent anti-CD3 antibody during the manufacturing process of allogeneic CAR T cells can markedly reduce graft-versus-host disease-related adverse effects associated with allogeneic CAR T cell therapy.

**Example 4. Selective Depletion of Activated T-Cells in Peripheral Blood by Anti-CD3 Antibody**

Through the foregoing experimental examples, it was confirmed that the monovalent anti-CD3 antibody of the present invention can prevent or treat graft-versus-host disease-related adverse effects by depleting residual TCR-positive cells during the manufacturing process of allogeneic CAR T cells.

In addition, the monovalent anti-CD3 antibody of the present invention has the potential to be used as a therapeutic agent by directly depleting activated T-cell populations in vivo when administered to patients suffering from inflammatory diseases mediated by activated T cells. Examples of such diseases include autoimmune diseases, graft-versus-host disease, and organ transplant rejection. That is, when the monovalent anti-CD3 antibody is administered to patients exhibiting autoimmune diseases, graft-versus-host disease, or organ transplant rejection, if activated T cells mediating inflammation can be selectively depleted, inflammatory responses mediated by T cells can be suppressed while preserving naïve T cells and memory T cells, thereby minimizing the risk of infection and enabling use of the antibody as an immunosuppressive agent.

Accordingly, it was investigated whether activated T-cell populations among human peripheral blood cells could be selectively depleted by treatment with the anti-CD3 scFv antibody (OKT3-scFv) of the present invention.

Specifically, T-cell populations within peripheral blood mononuclear cells (PBMCs) collected from healthy donors were classified into subpopulations based on activation history using expression of CD45RA and CCR7 as markers. The CD45RA(+)CCR7(+) population consists predominantly of naïve T cells that have not previously been activated, with a minor fraction of quiescent stem cell memory T cells (N/SCM). The CD45RA(-)CCR7(+) population corresponds to central memory T cells (CM) that have previously been activated but are currently in a quiescent state. These cell populations are therefore considered non-activated T-cell populations at present. In contrast, the CCR7(-) population is composed of effector T cells and effector memory T cells (EF/EM) that are activated or readily activatable. Accordingly, the EF/EM population contains a substantial proportion of currently activated T cells.

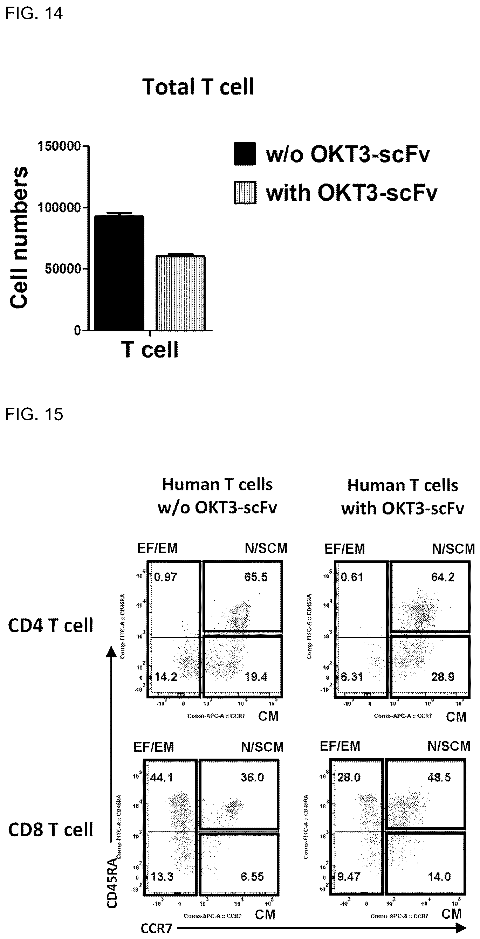

Peripheral blood mononuclear cells from healthy donors were cultured with the OKT3-scFv antibody for 3 days, after which changes in the proportions and absolute numbers of non-activated and activated T-cell subpopulations within the PBMCs were analyzed by flow cytometry. More specifically, during flow cytometric analysis, cell counting beads (Thermo Scientific) were added to determine the number of cells passing through the flow cytometer per unit volume, thereby enabling calculation of absolute numbers of total T cells and each T-cell subpopulation. Cells were stained with anti-CD3-PE, anti-CD4-APC-Cy7, anti-CD8-PE-Cy7, anti-CD45RA-FITC, anti-CCR7-APC, and 7-AAD. After gating on 7-AAD-negative, CD3-positive cells, the fractions of each T-cell subpopulation within CD4-positive and CD8-positive T cells were analyzed. The results are shown in FIGS. 14 to 16.

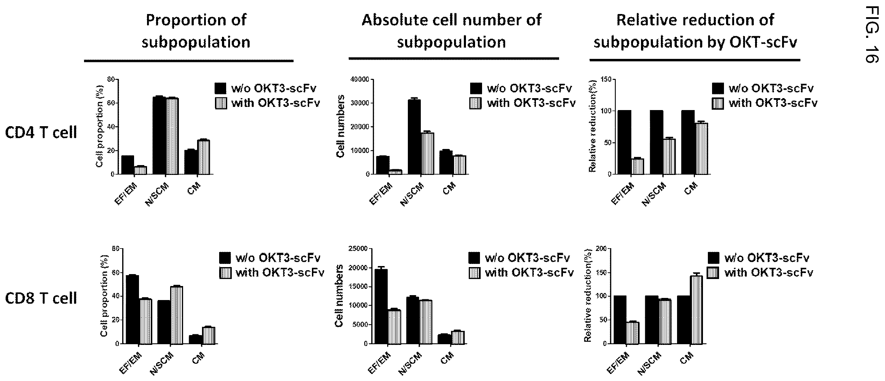

As shown in FIGS. 14 to 16, treatment with the OKT3-scFv antibody for 3 days resulted in an approximately 30% reduction in the total number of T cells (FIG. 14). Among the T-cell subpopulations, the proportion of the EF/EM population (activated T cells) was partially reduced compared to the N/SCM and CM populations (non-activated T cells) in both CD4 and CD8 T cells (FIGS. 15 and 16).

In addition, when absolute cell numbers of each subpopulation were calculated, CD4 T cells showed some reduction in the numbers of N/SCM and CM populations (non-activated T cells) following antibody treatment; however, the reduction in the EF/EM population (activated T cells) was much more pronounced. In CD8 T cells, little to no change in the numbers of N/SCM and CM populations (non-activated T cells) was observed following antibody treatment, whereas a marked reduction in the EF/EM population (activated T cells) was observed. Furthermore, when calculated as the ratio of cell number reduction in the antibody-treated group relative to the untreated group, the relative reduction rate of the EF/EM population (activated T cells) was significantly higher in both CD4 and CD8 T cells (FIG. 16).

From these results, it was confirmed that the monovalent anti-CD3 antibody exerts a selective depletion effect on activated T-cell populations among peripheral blood T cells, thereby demonstrating its utility as a multipurpose selective activated T-cell depleting agent and as a T-cell immunosuppressive agent.

### Example 5. *In Vivo* Depletion of Activated T Cells by Anti-CD3 Antibody

In order to examine whether depletion of activated T cells could also be observed upon direct in vivo administration of the monovalent anti-CD3 antibody, human T cells activated in vitro were administered to immunodeficient mice, followed by multiple intravenous administrations of the monovalent anti-CD3 antibody (UCHT1-scFv) at two-day intervals, and death of the human T cells was longitudinally monitored. In this experiment, human T cells engineered to express a luciferase enzyme were used, thereby enabling tracking of in vivo bioluminescence of the human T cells using an in vivo imaging system (IVIS).

Specifically, to generate luciferase-expressing human T cells, a high-sensitivity luciferase (eff-Luc) cDNA obtained from pDONR222-eGFP (Addgene plasmid #364493) was cloned into a partially modified pCDH-EF1 vector (Addgene plasmid #72266) so as to be co-expressed with GFP cDNA. The cloned lentiviral plasmid was co-transfected into a 293T cell line (ATCC) together with three packaging plasmids (pMD.2G, pMDLg/pRRE, and pRSV-rev) using Lipofectamine 3000 (Invitrogen). Culture supernatants containing lentiviral particles secreted for 24 and 48 hours were collected, filtered through a 0.45 $\mu$m filter to remove residual cellular debris, and concentrated 100-fold by ultracentrifugation to obtain a concentrated lentiviral preparation for transduction.

Leukocytes obtained from healthy donors by leukapheresis were added to a 24-well plate coated with an anti-CD3 antibody (OKT3, 10 $\mu$g/mL, BioXcell) together with an anti-CD28 antibody (CD28.2, 2 $\mu$g/mL, BD Biosciences), and cultured for 48 hours to activate T cells. Thereafter, a concentrated lentiviral preparation for luciferase expression was added to the T-cell culture medium and co-cultured with the cells. Two days after lentiviral transduction, the cells were washed to remove residual virus in the culture medium and further cultured by adding a culture medium containing human IL-2 (200 U/mL) twice at 3-day intervals (3 days + 3 days), thereby completing a 10-day protocol.

Because luciferase-expressing T cells enable in vivo monitoring of the presence and expansion of T cells using IVIS, $5 \times 10^6$ luciferase-expressing human T cells were intravenously injected into immunodeficient NSG mice. After human T-cell injection, the distribution of luciferase-expressing T cells in vivo was monitored at weekly intervals using an in vivo imaging system. Starting 14 days after T-cell injection (when T cells were detectable in all mice by IVIS), a monovalent anti-CD3 antibody (UCHT1-scFv; low dose: 20 $\mu$g per injection; high dose: 100 $\mu$g per injection) diluted in 200 $\mu$L of PBS was intravenously administered once every two days for a total of five administrations. From the time of initiation of monovalent anti-CD3 antibody administration, the distribution of T cells in vivo was monitored every two days using IVIS. For imaging, each mouse was intraperitoneally injected with 2 mg of luciferin dissolved in 100 $\mu$L of saline, and images were acquired by IVIS 10 minutes after injection.

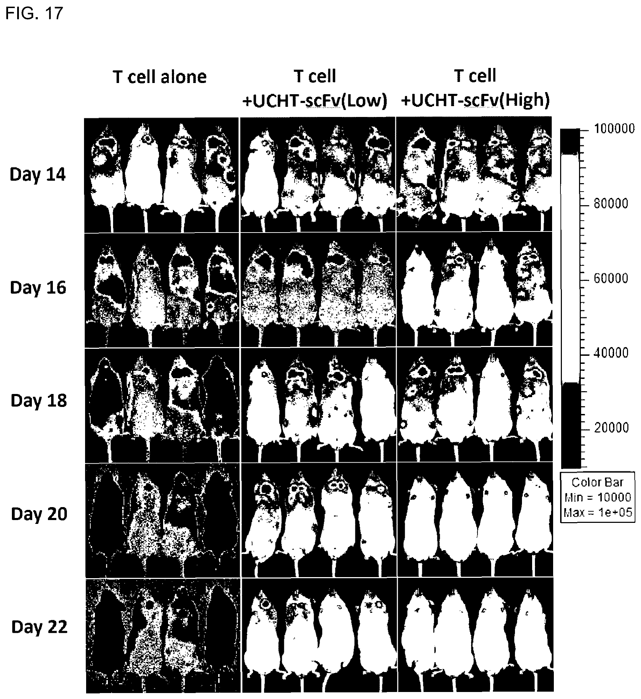

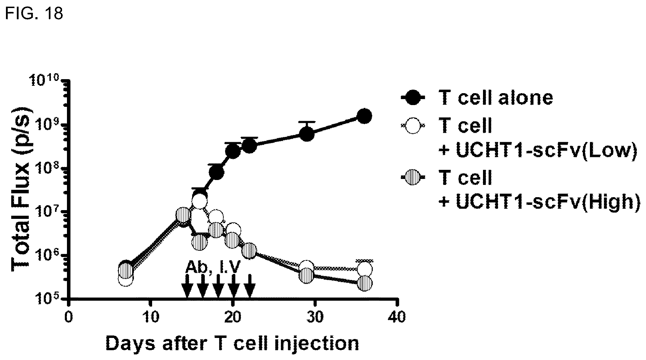

The experimental results are shown in FIGS. 17 and 18. From these results, it was confirmed that in vivo T cells were markedly depleted in the groups administered with the monovalent anti-CD3 antibody, and that depletion of T cells occurred more rapidly and more profoundly as the administered dose of the monovalent anti-CD3 antibody increased, thereby demonstrating a dose-dependent effect.

### Example 6. *In Vivo* Suppression of T Cell-Mediated Disease by Anti-CD3 Antibody

When luciferase-expressing human T cells are administered to immunodeficient mice, the human T cells attack normal mouse tissues and induce inflammation, thereby causing xenogeneic graft-versus-host disease (xeno-GVHD). Accordingly, it was examined whether administration of a monovalent anti-CD3 antibody could suppress xenogeneic graft-versus-host disease through depletion of human T cells *in vivo.*

The severity of graft-versus-host disease in mice was evaluated using two parameters: body weight and graft-versus-host disease (GVHD) score. Body weight and GVHD scores were measured twice per week. The GVHD score was calculated as the sum of scores assigned on a scale of 0 to 3 for each of five clinical parameters, including skin condition, fur condition, posture, activity level, and inflammatory eye disease.

FIG. 19 shows results of evaluating body weight loss, clinical symptoms of graft-versus-host disease, and mortality upon administration of a monovalent anti-CD3 antibody (UCHT1-scFv) after induction of xenogeneic graft-versus-host disease by human T cells in an immunodeficient mouse model. As shown in FIG. 19, it was confirmed that treatment with the monovalent anti-CD3 antibody markedly reduced body weight loss, clinical symptoms, and mortality caused by graft-versus-host disease. Accordingly, since the monovalent anti-CD3 antibody can substantially suppress inflammatory diseases induced by T cells, it has a high potential for development as a therapeutic agent for autoimmune diseases, which are similar T cell-mediated inflammatory diseases. In addition, as a disease analogous to graft-versus-host disease in this model, the monovalent anti-CD3 antibody may also be applicable for treatment of allogeneic graft-versus-host disease occurring due to donor T cells after allogeneic hematopoietic stem cell transplantation for treatment of hematologic malignancies.

In order to examine whether the monovalent anti-CD3 antibody has potential for use as a therapeutic agent to suppress organ transplant rejection mediated by recipient T cells after organ transplantation, an allogeneic mouse skin transplant model was employed. Specifically, skin from an allogeneic strain of C57BL6 mice was transplanted onto immunodeficient NSG mice, and activated human T cells were administered to induce immune rejection. Thereafter, it was evaluated whether graft rejection occurred, as indicated by impaired engraftment of the transplanted skin tissue.

Specifically, tail skin tissues ($1 \times 1 \times 0.5$ cm$^3$) derived from female C57BL/6 mice were transplanted onto the tail region of female immunodeficient NSG mice. The donor tail skin tissues were stored in PBS solution for up to 30 minutes prior to transplantation. The NSG mice were anesthetized throughout the surgical procedure using Avertin (8-10 mg in 400 μL PBS, intraperitoneal injection), and skin transplantation was then performed. Three pieces of donor skin were transplanted onto the tail of each NSG mouse. Following transplantation, the grafted sites were secured with a glass tube and adhesive bandage for 3 days to protect the transplanted skin. Graft survival was evaluated by daily visual inspection of the transplanted skin. Graft rejection was defined as necrosis of 75% or more of the surface area of the transplanted skin.

Fourteen days after tail skin transplantation, $2 \times 10^6$ activated human T cells were intravenously administered. A monovalent anti-CD3 antibody (UCHT1-scFv, 100 μg per dose, intraperitoneal injection) diluted in 200 μL PBS was administered starting on the day of T cell injection, once every two days for a total of four administrations as a first dosing cycle. After a 6-day rest period, a second dosing cycle was performed in the same manner as the first cycle, resulting in a total of eight administrations of the monovalent anti-CD3 antibody. Graft survival was calculated according to the following formula:

Graft survival (%)= Number of transplanted skin grafts remaining on the observation day/Number of transplanted skin grafts remaining on the day of T cell injection (14 days after skin transplantation) $\times$ 100.

FIG. 20 shows the results of evaluating whether graft rejection characterized by loss of transplanted skin tissue occurs in an immunodeficient mouse model upon administration of a monovalent anti-CD3 antibody (UCHT1-scFv) in the presence of human T cells. As shown in Figure 20, graft rejection in which most of the transplanted skin tissue was shed was observed in the group administered with human T cells alone, whereas it was confirmed that the engraftment rate of the transplanted skin was significantly improved in the group co-administered with the monovalent anti-CD3 antibody. Accordingly, the monovalent anti-CD3 antibody is useful as an immunosuppressive agent for inhibiting graft rejection in organ transplantation.

The foregoing description of the present invention is provided for illustrative purposes only, and those skilled in the art to which the present invention pertains will readily understand that various modifications and variations may be made thereto without departing from the technical spirit or essential characteristics of the present invention. Accordingly, the embodiments described herein are to be considered in all respects as illustrative and not restrictive.

**Claims**

1. A pharmaceutical composition for preventing or treating T cell-mediated autoimmune diseases, graft-versus-host diseases, or organ transplant rejection, comprising a monovalent antibody or a monovalent antigen-binding fragment thereof that specifically binds to CD3, wherein the monovalent antibody comprises a heavy chain variable region and a light chain variable region.

2. The pharmaceutical composition according to claim 1, wherein the antigen-binding fragment is Fab, Fab', Fv, scFv, or single-domain antibody (sdAb).

3. The pharmaceutical composition according to claim 1, wherein the antibody that specifically binds to CD3 is selected from the group consisting of OKT3, UCHT1, Teplizumab, Otelixizumab, Visilizumab, Foralumab; an antibody comprising a light chain variable region comprising LCDR1 of SEQ ID NO: 23, LCDR2 of SEQ ID NO: 24, and LCDR3 of SEQ ID NO: 25, and a heavy chain variable region comprising HCDR1 of SEQ ID NO: 26, HCDR2 of SEQ ID NO: 27, and HCDR3 of SEQ ID NO: 28; and an antibody comprising a light chain variable region comprising LCDR1 of SEQ ID NO: 32, LCDR2 of SEQ ID NO: 33, and LCDR3 of SEQ ID NO: 34, and a heavy chain variable region comprising HCDR1 of SEQ ID NO: 35, HCDR2 of SEQ ID NO: 36, and HCDR3 of SEQ ID NO: 37.

4. The pharmaceutical composition according to claim 1, wherein the antibody that specifically binds to CD3 is selected from the group consisting of teclistamab, tebentafusp, blinatumomab, catumaxomab, TNB-486, AMG562, duvortuxizumab, AMG910, pasotuxizumab, HPN424, AMG 160, JNJ-63898081, CC-1, AMG 509, HPN536, odronextamab, epcoritamab, glofitamab, mosunetuzumab, JNJ-75348780, vixtimotamab, AMG 330, REGN4018, AMG199, MGD007, EGFR BAT, AMG596, M701, solitomab, MT110, AMG110, AMG 211, MEDI-565, cibisatamab, tidutamab, talquetamab, RG6194, GBR 1302, M802, runimotamab, GEN1044, GEN1047, PF-07062119, AMG 757, BI 764532, HPN328, Hu3F8-BsAb, GEM3PSCA, IMC-C103C, IMC-F106C, JNJ-70218902, AMG 424, elranatamab, ABBV-383, AMG 420, CC-93269, linvoseltamab, alnuctamab, cevostamab, and AMG 427, wherein the heavy chain variable region and the light chain variable region target CD3.

5. The pharmaceutical composition according to claim 1, wherein the monovalent antibody or monovalent antigen-binding fragment thereof induces death of activated T cells and/or suppresses alloreactive immune responses.

6. The pharmaceutical composition according to claim 1, wherein the autoimmune disease is selected from the group consisting of rheumatoid arthritis, multiple sclerosis, systemic lupus erythematosus, type 1 diabetes, Crohn's disease, scleroderma, Sjogren's syndrome, psoriasis, inflammatory bowel disease, ulcerative colitis, ankylosing spondylitis, interstitial lung disease, uveitis, optic neuritis, peripheral neuropathies, sarcoidosis, antiphospholipid syndrome, inflammatory myopathies, Behcet's disease, alopecia totalis, alopecia universalis, pemphigus vulgaris, myasthenia gravis, Graves' disease, Hashimoto's thyroiditis, Guillain-Barre syndrome, celiac disease, and pernicious anemia.

7. The pharmaceutical composition according to claim 1, which is used in combination with an immunosuppressive agent.

8. A pharmaceutical composition for suppressing T-cell immunity or depleting activated T cells, comprising a monovalent antibody or a monovalent antigen-binding fragment thereof that specifically binds to CD3, wherein the monovalent antibody comprises a heavy chain variable region and a light chain variable region.

9. A method for suppressing T-cell immunity, comprising administering a monovalent antibody or a monovalent antigen-binding fragment thereof that specifically binds to CD3, wherein the monovalent antibody comprises a heavy chain variable region and a light chain variable region.

10. A method for depleting TCR (T cell receptor)-positive CAR-T cells, comprising:

    (a) down-regulating expression of a T cell receptor (TCR) in T cells;
    (b) introducing a chimeric antigen receptor (CAR) into the T cells; and
    (c) treating the cells obtained in steps (a) and (b) with a monovalent antibody or a monovalent antigen-binding fragment thereof that specifically binds to CD3, wherein the monovalent antibody comprises a heavy chain variable region and a light chain variable region,

    wherein steps (a) and (b) are performed in any order.

11. The method according to claim 10, wherein the step of downregulating expression of the TCR is performed using a genome editing technique, siRNA, shRNA, or miRNA.

12. The method according to claim 11, wherein the genome editing technique is performed using CRISPR (clustered regularly interspaced short palindromic repeats)/Cas9, TALEN, a zinc finger nuclease, base editing, or prime editing.

13. An antibody or an antigen-binding fragment thereof, comprising:

    a light chain variable region comprising LCDR1 of SEQ ID NO: 23, LCDR2 of SEQ ID NO: 24, and LCDR3 of SEQ ID NO: 25, and a heavy chain variable region comprising HCDR1 of SEQ ID NO: 26, HCDR2 of SEQ ID NO: 27, and HCDR3 of SEQ ID NO: 28; or
    a light chain variable region comprising LCDR1 of SEQ ID NO: 32, LCDR2 of SEQ ID NO: 33, and LCDR3 of SEQ ID NO: 34, and a heavy chain variable region comprising HCDR1 of SEQ ID NO: 35, HCDR2 of SEQ ID NO: 36, and HCDR3 of SEQ ID NO: 37.

14. The antibody or antigen-binding fragment thereof according to claim 13, comprising a light chain variable region comprising the amino acid sequence of SEQ ID NO: 29 or 38 and/or a heavy chain variable region comprising the amino acid sequence of SEQ ID NO: 30 or 39.

15. A polynucleotide encoding the antibody or antigen-binding fragment thereof according to claim 13.

16. An expression vector comprising the polynucleotide according to claim 15.

17. A cell comprising the polynucleotide according to claim 15 or the expression vector comprising the polynucleotide.

FIG. 1

FIG. 2

░ Ab untreated
▒ Ab treated

Antibody :
(12hr)

| Ctrl | OKT3-IgG | OKT3-Fab | OKT3-scFv |

Active Caspase 3

- ● Act T cell
- □ Act T cell + OKT3-IgG
- △ Act T cell + OKT3-Fab
- ○ Act T cell + OKT3-scFv

FIG. 3

FIG. 4

FIG. 5

FIG. 6

FIG. 7

a

b

FIG. 8

FIG. 9

FIG. 10

T cells
stimulation

TCR deletion
&
CAR Transduction

Depleting
Antibody
(OKT3-scFv)

FACS
analysis

Day    0              2                    7         10

Day 7
Before Ab treatment

Day 10
After Ab treatment

CAR

TCRαβ

FIG. 11

FIG. 12

FIG. 13

FIG. 14

## Total T cell

FIG. 15

FIG. 16

FIG. 17

FIG. 18

FIG. 19

FIG. 20

## INTERNATIONAL SEARCH REPORT

| International application No. |
|---|
| **PCT/IB2024/054911** |

| A. | CLASSIFICATION OF SUBJECT MATTER |
|---|---|

**C07K 16/28**(2006.01)i; **A61P 37/06**(2006.01)i; **C12N 15/85**(2006.01)i; **A61K 39/00**(2006.01)i

According to International Patent Classification (IPC) or to both national classification and IPC

| B. | FIELDS SEARCHED |
|---|---|

Minimum documentation searched (classification system followed by classification symbols)

C07K 16/28(2006.01); A61K 35/17(2015.01); A61P 37/00(2006.01); C07K 16/46(2006.01); C12N 15/85(2006.01); C12N 5/0783(2010.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: CD3, T 세포 수용체(T cell receptor, TCR), 키메릭 항원 수용체(chimeric antigen receptor, CAR), OKT3, 항체(antibody), 단일-사슬 가변 단편(single-chain variable fragment, scFv), 유전자 편집(gene editing)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | KR 10-2020-0035304 A (NATIONAL UNIVERSITY OF SINGAPORE) 02 April 2020 (2020-04-02)<br>See paragraphs [0011]-[0269]. | 1-8,10-12 |
| A | | 13-17 |
| A | US 2020-0087398 A1 (UCL BUSINESS PLC) 19 March 2020 (2020-03-19)<br>See paragraphs [0007], [0008], [0064], [0072]-[0076] and [0133]. | 1-8,10-17 |
| A | US 2020-0332004 A1 (CELLECTIS) 22 October 2020 (2020-10-22)<br>See claim 1. | 1-8,10-17 |
| A | KR 10-2014-0091038 A (TOLERA THERAPEUTICS, INC.) 18 July 2014 (2014-07-18)<br>See abstract. | 1-8,10-17 |
| A | US 2019-0263904 A1 (TENEOBIO, INC.) 29 August 2019 (2019-08-29)<br>See entire document. | 1-8,10-17 |

☐ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **24 September 2024** | **25 September 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
|---|
| **PCT/IB2024/054911** |

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/IB2024/054911** |

**Box No. II        Observations where certain claims were found unsearchable (Continuation of item 2 of first sheet)**

This international search report has not been established in respect of certain claims under Article 17(2)(a) for the following reasons:

1. ☑ Claims Nos.: **9**
   because they relate to subject matter not required to be searched by this Authority, namely:

   Claim 9 pertains to a method for treatment of the human body by therapy (PCT Article 17(2)(a)(i) and PCT Rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

| International application No. |
|---|
| **PCT/IB2024/054911** |

| Patent document cited in search report | | | Publication date (day/month/year) | Patent family member(s) | | | Publication date (day/month/year) |
|---|---|---|---|---|---|---|---|
| KR | 10-2020-0035304 | A | 02 April 2020 | CN | 111247241 | A | 05 June 2020 |
| | | | | EP | 3665270 | A1 | 17 June 2020 |
| | | | | JP | 2020-530291 | A | 22 October 2020 |
| | | | | US | 2019-0038733 | A1 | 07 February 2019 |
| | | | | US | 2019-0046571 | A1 | 14 February 2019 |
| | | | | US | 2023-0364140 | A1 | 16 November 2023 |
| | | | | WO | 2019-032916 | A1 | 14 February 2019 |
| US | 2020-0087398 | A1 | 19 March 2020 | EP | 3559215 | A1 | 30 October 2019 |
| | | | | WO | 2018-115906 | A1 | 28 June 2018 |
| US | 2020-0332004 | A1 | 22 October 2020 | EP | 3684399 | A1 | 29 July 2020 |
| | | | | JP | 2021-508456 | A | 11 March 2021 |
| | | | | JP | 7372920 | B2 | 01 November 2023 |
| | | | | WO | 2019-129851 | A1 | 04 July 2019 |
| KR | 10-2014-0091038 | A | 18 July 2014 | AU | 2012-332193 | A1 | 15 May 2014 |
| | | | | AU | 2012-332193 | A1 | 10 May 2013 |
| | | | | BR | 112014010532 | A2 | 18 April 2017 |
| | | | | CN | 104870011 | A | 26 August 2015 |
| | | | | EP | 2773670 | A2 | 10 September 2014 |
| | | | | JP | 2015-504419 | A | 12 February 2015 |
| | | | | US | 2013-0302301 | A1 | 14 November 2013 |
| | | | | US | 2013-0330351 | A1 | 12 December 2013 |
| | | | | US | 2015-0252109 | A1 | 10 September 2015 |
| | | | | WO | 2013-067517 | A2 | 10 May 2013 |
| US | 2019-0263904 | A1 | 29 August 2019 | CN | 109843325 | B | 03 October 2023 |
| | | | | CN | 114395048 | A | 26 April 2022 |
| | | | | CN | 117603353 | A | 27 February 2024 |
| | | | | EP | 3471773 | A1 | 24 April 2019 |
| | | | | EP | 3512549 | A1 | 24 July 2019 |
| | | | | EP | 4417263 | A2 | 21 August 2024 |
| | | | | JP | 7030109 | B2 | 04 March 2022 |
| | | | | JP | 7321303 | B2 | 04 August 2023 |
| | | | | JP | 7431868 | B2 | 15 February 2024 |
| | | | | KR | 10-2023-0035706 | A | 14 March 2023 |
| | | | | KR | 10-2023-0129583 | A | 08 September 2023 |
| | | | | KR | 10-2024-0044520 | A | 04 April 2024 |
| | | | | US | 11421027 | B2 | 23 August 2022 |
| | | | | US | 11505606 | B2 | 22 November 2022 |
| | | | | US | 2020-0048348 | A1 | 13 February 2020 |
| | | | | WO | 2017-223111 | A1 | 28 December 2017 |
| | | | | WO | 2018-052503 | A1 | 22 March 2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 8810649 A
- WO 88106630 A
- WO 8807085 A
- WO 8807086 A
- WO 8809344 A
- US 20130287748 A1

**Non-patent literature cited in the description**

- **PEARSON et al.** *Proc. Natl. Acad. Sci. USA*, 1988, vol. 85, 2444
- **NEEDLEMAN** ; **WUNSCH**. *J. Mol. Biol.*, 1970, vol. 48, 443-453
- **RICE et al.** EMBOSS: The European Molecular Biology Open Software Suite. *Trends Genet.*, 2000, vol. 16, 276-277
- **DEVEREUX, J. et al.** *Nucleic Acids Research*, 1984, vol. 12, 387
- **ALTSCHUL, S. F. et al.** *J Molec Biol*, 1990, vol. 215, 403
- Guide to Huge Computers. Academic Press, 1994
- **CARRILLO et al.** *SIAM J Applied Math*, 1988, vol. 48, 1073
- *Mol. Ther. Methods Clin. Dev.*, 2016, vol. 3, 16017
- *J. Virol. Methods*, vol. 2004, 122-131
- *Therapy-Methods & Clinical Development*, 2016, vol. 3, 16017
- *Mol. Ther.*, March 2016, vol. 24 (3), 570-581